# EUROPEAN PATENT SPECIFICATION

(11) **EP 3 207 158 B1**
(45) Date of publication and mention of the grant of the patent: **21.08.2019**
(21) Application number: 15790674.4
(22) Date of filing: 15.10.2015
(51) Int. Cl.: C12Q 1/6886

(54) **GLYCOSYLTRANSFERASE GENE EXPRESSION PROFILE TO IDENTIFY MULTIPLE CANCER TYPES AND SUBTYPES**
GLYCOSYLTRANSFERASE-GENEXPRESSIONSPROFILS ZUR IDENTIFIZIERUNG VON MEHREREN KREBSARTEN UND -UNTERARTEN
PROFIL D'EXPRESSION DU GÈNE DE LA GLYCOSYLTRANSFÉRASE POUR IDENTIFIER DE MULTIPLES TYPES ET SOUS-TYPES DE CANCERS

(30) Priority: 15.10.2014 GB 201418242
(43) Date of publication of application: 23.08.2017
(73) Proprietor: University Of Cape Town, 7701 Cape Town (ZA)
(72) Inventor: NAIDOO, Kevin Jonathan, 7708 Claremont (ZA); ASHKANI, Jahanshah, 7500 Plattekloof (ZA)
(74) Representative: Docherty, Robert Charles
(86) International application number: PCT/IB2015/057916
(87) International publication number: WO 2016/059585

(56) References cited:
- WO-A1-2013/152301
- US-A1- 2013 064 901
- POTAPENKO I O ET AL: "Glycan gene expression signatures in normal and malignant breast tissue; possible role in diagnosis and progression", MOLECULAR ONCOLOGY, ELSEVIER, AMSTERDAM, NL, vol. 4, no. 2, 1 April 2010 (2010-04-01), pages 98-118, XP026997744, ISSN: 1574-7891 [retrieved on 2009-12-11]
- PETRETTI T ET AL: "Altered mRNA expression of glycosyltransferases in human colorectal carcinomas and liver metastases", GUT, BRITISH MEDICAL ASSOCIATION, LONDON, UK, vol. 46, no. 3, 1 January 2000 (2000-01-01), pages 359-366, XP008108243, ISSN: 0017-5749
- "GeneChip Human Genome U133 set", ANNOUNCEMENT AFFYMETRIX, XX, XX, 1 September 2001 (2001-09-01), XP002324895,
- TIBSHIRANI R ET AL: "Diagnosis of multiple cancer types by shrunken centroids of gene expression", PROCEEDINGS OF THE NATIONAL ACADEMY OF SCIENCES, NATIONAL ACADEMY OF SCIENCES, US, vol. 99, no. 10, 14 May 2002 (2002-05-14), pages 6567-6572, XP002988576, ISSN: 0027-8424, DOI: 10.1073/PNAS.082099299
- HAMMOND JENNIFER A ET AL: "Expression profiling of glycosyltransferases and related enzymes using gene microarrays", 2006, GLYCOMICS; [METHODS IN ENZYMOLOGY; ISSN 0076-6879; VOL. 416], PAGE(S) 141 - 156, XP008178595, ISBN: 978-0-12-182821-9 [retrieved on 2006-11-17] abstract

## Description

### FIELD OF THE INVENTION

This invention relates to a method of identification of cancer types in a subject comprising the use of supervised algorithms that have been trained to evaluate quantified glycosyltransferase gene expression in a sample from the subject. In addition, the method relates to a method of cancer subtype classification and/or identification of a cancer subtype within a specific cancer type comprising the use of unsupervised and supervised algorithms that have been trained to evaluate quantified glycosyltransferase gene expression. The invention further relates to a kit comprising glycosyltransferase gene capture probes or primers for use with the method, and a method of treatment of cancer comprising the use of the method and/or kit of the invention to select an appropriate treatment regime for a subject and/or to monitor the response of the subject to the cancer treatment.

### BACKGROUND OF THE INVENTION

Cancer is one of the most dreadful diseases of this century, which can be life threatening. Early diagnosis and treatment relevant to the cancer type may improve the prognosis for cancer patients. The molecular characteristic of each type or subtype of cancer affects its sensitivity to treatments, which signifies the importance of precise prognosis in maximizing the efficacy and minimizing the toxicity of treatments. Despite the impact of the use of global gene expression analysis by genomic and epigenomic studies using high-throughput technologies, in elucidating the molecular characteristics of cancer, a broad diversity is often observed in biological behaviour, clinical prognosis, sensitivity to treatments, and therapeutic targets. This highlights the importance of characterising these possible subclasses within a cancer type. Such classification in the context of gene expression may be achievable through identification of key drivers in cancer biology.

Tumour cells have an altered cellular biochemistry that facilitates evasion of growth suppressors and apoptosis as well as acquire invasive, angiogenic and metastatic properties. More pointedly it is unclear to what extent the degree of specific glycosylation mechanisms interface with these pathways. It is known that changes occur in glycans and including glycans on proteins and lipids in and on cancer cells, making these altered glycans promising candidates for use as cancer biomarkers that may be used for early diagnosis, monitoring and therapy in a cancer patient. However, due to the lack of suitable analytical methods for glycan-biomarker identification and detection most of the existing cancer markers are the glycosylated proteins themselves that are not recommended for early detection because of poor sensitivity and specificity.

Furthermore, despite progress in feature selection algorithms used in different cancer subtyping methods, the identification of driver genes involved in different types of cancers remains challenging. Therefore, the development of novel strategies are significantly needed for improved early diagnosis and efficient cancer therapy.

Document US 2013/064901 discloses a method of identification of cancer type (subtyping of gastric cancer) comprising detecting expression markers characteristic of said cancer types. Document WO 2013/152301 discloses expression profiles of genes involved in glycan metabolism in association with ovarian cancer.

### SUMMARY OF THE INVENTION

We disclose a method of identification of cancer type in a subject by obtaining a glycosyltransferase gene expression profile from a set of glycosyltransferase genes in a test sample from the subject and comparing the gene expression profile from the test sample to a reference set of glycosyltransferase gene expression centroids from known cancer types, wherein the subject's cancer type is identified as the cancer type corresponding to the nearest reference set glycosyltransferase gene centroid compared with the glycosyltransferase gene expression profile from the test sample.

According to an aspect of the invention there is provided an in vitro method of identification of cancer type from the group consisting of six cancer types selected from breast invasive carcinoma, lung squamous cell carcinoma, ovarian serous cystadenocarcinoma cancer, glioblastoma multiforme, colon adenocarcinoma, and kidney renal clear cell carcinoma in a test sample from a subject comprising the steps of:
(a) obtaining a glycosyltransferase gene expression profile from a set of 210 glycosyltransferase genes consisting of those listed in Table 1 in the test sample from the subject;
(b) calculating the distance of the gene expression profile of the test sample to a reference set of glycosyltransferase gene expression centroids from the six cancer types; and
assigning the test sample to a cancer type, wherein the cancer type is identified as the cancer type corresponding to the nearest reference set glycosyltransferase gene centroid of the six cancer types glycosyltransferase gene expression centroids compared with the glycosyltransferase gene expression profile from the test sample

The method may comprise the following steps:
(a) obtaining a glycosyltransferase gene expression profile from a set of glycosyltransferase genes in a test sample from the subject, wherein the glycosyltransferase genes are selected from the group consisting of the glycosyltransferase genes listed in Table 1;
(b) calculating the distance of the gene expression profile of the test sample to each of a set of reference glycosyltransferase gene expression centroids from known cancer types; and
(c) assigning the test sample to a cancer type wherein the cancer type is determined as that corresponding to the nearest reference set glycosyltransferase gene centroid compared with the glycosyltransferase gene expression profile from the test sample.

The method may further comprise the following steps:
(a) obtaining a glycosyltransferase gene expression profile for each of a training set of cancer samples from subjects with known cancer types, wherein the glycosyltransferase genes are selected from the group consisting of the glycosyltransferase genes listed in Table 1;
(b) using a supervised algorithm to construct gene expression centroids for each of the cancer types in the training set;
(c) obtaining a glycosyltransferase gene expression profile from a set of glycosyltransferase genes in a test sample from the subject, wherein the glycosyltransferase genes are selected from the group consisting of the glycosyltransferase genes listed in Table 1;
(d) calculating the distance of the gene expression profile of the test sample to each of glycosyltransferase gene expression centroids from the training set of known cancer types; and
(e) assigning the test sample to a cancer type wherein the cancer type is determined as that corresponding to the nearest training set glycosyltransferase gene centroid compared with the glycosyltransferase gene expression profile from the test sample.

The glycosyltransferase genes may consist of any 5 or more, any 10 or more, any 20 or more, any 30 or more, any 40 or more, any 50 or more, any 60 or more, any 63 or more, any 70 or more, any 80 or more, any 90 or more, any 100 or more, any 110 or more any 120 or more any 130 or more, any 140 or more any 150 or more, any 160 or more any 170 or more, any 177 or more, optionally any 178 or more, further optionally any 179 or more, still further optionally any 180 or more, 181 or more, 182 or more, 183 or more, 184 or more, 185 or more etc. and up to still further optionally all 210 of the glycosyltransferase genes listed in Table 1.

Preferably, the glycosyltransferase genes consist of all 210 of the glycosyltransferase genes listed in Table 1.

In particular, where all of the 210 glycosyltransferase genes are used, the method of the invention may be used to identify cancer types selected from breast, including breast invasive carcinoma, brain, including glioblastoma multiforme, colon, including colon adenocarcinoma, kidney, including kidney renal clear cell carcinoma, lung, including lung squamous cell carcinoma, and ovarian, including ovarian serous cystadenocarcinoma cancer.

Where at least 50 glycosyltransferase genes are used, the method of the invention may be used to identify cancer types selected from breast, including breast invasive carcinoma, brain, including glioblastoma multiforme, colon, including colon adenocarcinoma, and ovarian, including ovarian serous cystadenocarcinoma cancer.

Where at least 5 glycosyltransferase genes are used, the method of the invention may be used to identify cancer types selected from brain, including glioblastoma multiforme, and ovarian, including ovarian serous cystadenocarcinoma cancer.

The reference glycosyltransferase gene expression centroids or training set of glycosyltransferase genes from known cancer types, or both may comprise or optionally consist of cancer types selected from breast, including breast invasive carcinoma, brain, including glioblastoma multiforme, colon, including colon adenocarcinoma, kidney, including kidney renal clear cell carcinoma, lung, including lung squamous cell carcinoma, and ovarian, including ovarian serous cystadenocarcinoma cancer.

The sample may be any bodily sample, such as, but not limited to, a tissue, blood, plasma or serum sample.

Step (a) of the method is preferably performed with cDNA or mRNA amplified from RNA extracted from the sample. Many means of amplification of cDNA or mRNA from RNA templates are known to those skilled in the art and many commercial kits for RNA extraction and amplification of cDNA and mRNA are available.

The determining or quantification of expression levels in step (a) may be performed using a dot blot procedure well known to those skilled in the art, including but not limited to, membrane-based Northern blot or Southern blot, or by means of a miniaturised dot blot procedure such as a microarray. Many commercial kits for use in such procedures are available to those skilled in the art.

Alternatively, the quantification may be performed by means of a quantitative polymerase chain reaction (PCR) method. Different methods for quantitative PCR of both RNA and DNA are known to those skilled in the art and commercial kits for use in such procedures are available. Examples of such procedures include, but are not limited to, quantitative reverse transcriptase PCR (RT-qPCR) which may or may not be real time.

In one particular embodiment of the invention, the method may comprise the steps of:
(a) purifying total RNA from the sample of the subject;
(b) converting the RNA into cDNA by reverse transcription and labelling the cDNA with a fluorescent dye or amplifying mRNA from the RNA and labelling the mRNA with a fluorescent dye;
(c) contacting a microarray slide printed with target oligonucleotide probes specific for the 210 glycosyltransferase genes listed in table 1 or a gene fragment of each with the fluorescently labelled cDNA or mRNA;
(d) hybridizing the fluorescently labelled cDNA or mRNA to the target oligonucleotide probes;
(e) quantifying the intensity of fluorescence of cDNA or mRNA bound to their target oligonucleotide probes on the microarray slide, whereby the intensity of fluorescence at each target corresponds to the relative abundance of mRNA transcript for each subject glycosyltransferase gene, that is, the subject sample glycosyltransferase gene expression profile; and optionally,
(f) calculating the distance of the gene expression profile of the subject sample to each of a set of glycosyltransferase gene expression centroids from known cancer types, wherein the glycosyltransferase gene expression centroid genes are selected from the group consisting of the glycosyltransferase genes listed in Table 1; and
(g) assigning the subject sample to a cancer type wherein the cancer type is determined as that corresponding to the nearest glycosyltransferase gene expression centroid compared with the glycosyltransferase gene expression profile from the subject sample.

In an alternative particular embodiment of the invention, the method may comprise the steps of:
(a) purifying total RNA from the sample of the subject;
(b) converting the RNA into cDNA by reverse transcription and labelling the cDNA with a fluorescent dye or a chromogenic substrate, or amplifying mRNA from the RNA and labelling the mRNA with a fluorescent dye or a chromogenic substrate;
(c) contacting a dot blot printed with target oligonucleotide probes specific for the 210 glycosyltransferase genes listed in Table 1 or a gene fragment of each 1 with the labelled cDNA or mRNA;
(d) hybridizing the labelled cDNA or mRNA to the target oligonucleotide probes;
(e) quantifying the intensity of the hybridized labelled cDNA or mRNA bound to their target oligonucleotide probes on the dot blot, whereby the intensity of the label at each target corresponds to the relative abundance of mRNA transcript for each gene, that is, the subject sample glycosyltransferase gene expression profile; and optionally,
(f) calculating the distance of the gene expression profile of the subject sample to each of a set of glycosyltransferase gene expression centroids from known cancer types; and
(g) assigning the subject sample to a cancer type wherein the cancer type is determined as that corresponding to the nearest glycosyltransferase gene expression centroid compared with the glycosyltransferase gene expression profile from the subject sample.

In a further alternative particular embodiment of the invention, the method may comprise the steps of:
(a) purifying total RNA from the sample of the subject;
(b) amplifying cDNA from the RNA by RT-qPCR with specific primers for the 210 glycosyltransferase genes listed in table 1 or a gene fragment of each
(c) quantifying the amplified cDNA;
(d) assessing the relative abundance of the amplified cDNA of each of the genes, whereby the abundance of the amplified cDNA corresponds to the relative abundance of mRNA transcript for each gene, that is, the subject sample glycosyltransferase gene expression profile; and optionally,
(e) calculating the distance of the gene expression profile of the subject sample to each of a set of glycosyltransferase gene expression centroids from known cancer types; and
(f) assigning the subject sample to a cancer type wherein the cancer type is determined as that corresponding to the nearest glycosyltransferase gene expression centroid compared with the glycosyltransferase gene expression profile from the subject sample.

We disclose that the method may further comprise a step of classifying the subtype of the cancer in a subject with known cancer type, wherein the subtype classification comprises the steps of:
(a) obtaining a test glycosyltransferase gene expression profile from a cancer sample from a subject with known cancer type, wherein the glycosyltransferase genes are selected from the group consisting of the glycosyltransferase genes listed in Table 1;
(b) providing a reference set of glycosyltransferase gene expression profiles from subtypes of the corresponding cancer to that of the subject, wherein the glycosyltransferase genes are selected from the group consisting of the glycosyltransferase genes listed in Table 1;
(c) performing unsupervised clustering analysis on the test glycosyltransferase gene expression profile and the reference set of glycosyltransferase gene expression profiles, thereby grouping the test glycosyltransferase gene expression profile and the reference glycosyltransferase gene expression profiles into one or more subtypes.

Preferably, the cancer type of the subject may previously have been identified by means of the method of the invention.

Optionally, the method of subtyping may further comprise the following steps:
(d) obtaining a glycosyltransferase gene expression profile from a set of glycosyltransferase genes in a test sample from a subject with known cancer type, wherein the glycosyltransferase genes are selected from the group consisting of the glycosyltransferase genes listed in Table 1;
(e) calculating the distance of the gene expression profile of the test sample to each of a set of reference glycosyltransferase gene expression centroids from subtypes of the corresponding cancer to that of the subject; and
(f) assigning the test sample to a cancer subtype wherein the cancer subtype is determined as that corresponding to the nearest reference set glycosyltransferase gene centroid compared with the glycosyltransferase gene expression profile from the test sample.

Further optionally, the method may further comprise the following steps:
(f) obtaining a glycosyltransferase gene expression profile for each of a training set of cancer samples from subjects with a known cancer type, wherein the glycosyltransferase genes are selected from the group consisting of the glycosyltransferase genes listed in Table 1;
(g) using a supervised algorithm to construct gene expression centroids for each of the cancer types in the training set;
(h) obtaining a glycosyltransferase gene expression profile from a set of glycosyltransferase genes in a test sample from the subject, wherein the glycosyltransferase genes are selected from the group consisting of the glycosyltransferase genes listed in Table 1;
(i) calculating the distance of the gene expression profile of the test sample to each of glycosyltransferase gene expression centroids constructed in step (b); and
(j) assigning the test sample to a cancer subtype wherein the cancer subtype is determined as that corresponding to the nearest training set glycosyltransferase gene centroid compared with the glycosyltransferase gene expression profile from the test sample.

Survival information may be known for the reference or training set of glycosyltransferase gene expression profiles, in which case the method may optionally include a step of identifying the survival prognosis of the subject based on the survival prognosis of the subtype cluster within which the test glycosyltransferase gene expression profile groups.

In particular, the step of identifying the subtype of the cancer after identification of cancer type may be performed on samples from subjects identified as having breast, brain or ovarian cancer.

We further disclose
a kit for identifying cancer type and optionally subtype in a subject according to the method of the invention, the kit comprising:
(a) primers for amplification of cDNA or mRNA;
(b) glycosyltransferase gene specific oligonucleotide probes for hybridizing to cDNA or mRNA in a dot blot procedure; or
(c) glycosyltransferase gene specific oligonucleotide probes printed on a microarray slide, to which labelled cDNA or RNA can be hybridized in a microarray method;
(d) an indicator means; and
(e) optionally, directions for use.

According to an aspect of the invention there is provided a kit for classifying a cancer type selected from the group consisting of six cancer types selected from breast invasive carcinoma, lung squamous cell carcinoma, and ovarian serous cystadenocarcinoma cancer, glioblastoma multiforme, colon adenocarcinoma, and kidney renal clear cell carcinoma in a test sample from a subject and optionally also cancer subtype in the subject with the use of the method of the invention the kit comprising:
(a) primers for amplification of cDNA or mRNA;
(b) gene specific oligonucleotide probes for hybridizing to cDNA or mRNA in a dot blot procedure; or
(c) gene specific oligonucleotide probes printed on a microarray slide, to which labelled cDNA or RNA can be hybridized in a microarray method;
(d) an indicator means;
   wherein the primers or gene specific oligonucleotide probes are specific to the 210 glycosyltransferase genes or fragments thereof listed in Table 1;
(e) optionally, directions for use;
(f) optionally a reference set of glycosyltransferase polynucleotides from the six cancer types or subtypes thereof, wherein the polynucleotides comprise the glycosyltransferase genes listed in Table 1 or fragments thereof; and
(g) optionally computer readable instructions for any one or more of:
   A. determining the subject test sample glycosyltransferase gene expression profile;
   B. identifying cancer type by:
      i. calculating the distance of the gene expression profile of the subject test sample to each of the set of glycosyltransferase gene expression centroids from the six cancer types; and
      ii. assigning the subject test sample to the cancer type wherein the cancer type is determined as that corresponding to the nearest glycosyltransferase gene expression centroid compared with the glycosyltransferase gene expression profile from the subject test sample; and
   C. classifying and/or identifying the cancer subtype by:
      i. performing unsupervised consensus clustering analysis of the glycosyltransferase gene expression profile from the subject test sample having one of the six cancer types compared with the glycosyltransferase gene expression profiles from a set of reference or training glycosyltransferase genes from subtypes of the corresponding cancer to that of the subject;
      ii. calculating the distance of the gene expression profile of the subject test sample to each of the set of glycosyltransferase gene expression centroids from subtypes of the corresponding cancer to that of the subject; and
      iii. assigning the subject test sample to a cancer subtype wherein the cancer subtype is determined as that corresponding to the nearest glycosyltransferase gene expression centroid compared with the glycosyltransferase gene expression profile from the subject test sample.

The dot blot procedure used may be a cDNA or mRNA dot blot procedure. Preferably, the procedure is a miniaturised dot blot such as a microarray.

In an alternative embodiment of the invention, the kit comprises:
(a) glycosyltransferase gene specific primers for amplification of cDNA or mRNA by quantitative real-time PCR; and
(b) an indicator means; and
(c) optionally, directions for use.

The kit may further comprise a reference set of glycosyltransferase polynucleotides from known cancer types or subtypes in a specific cancer type.

The primers may be sequence-specific primers, oligo(dT) primers or random primers. For example, the sequence-specific primers may be glycosyltransferase gene or gene fragment specific primers. Various methods and websites for primer design and labelling are well known to those skilled in the art. Furthermore, methods and kits for amplification of cDNA or mRNA are also well known and available to those skilled in the art.

The oligonucleotide probes may be DNA, RNA or a modified or derivative oligonucleotide thereof, typically with a minimum of 25 complementary bases to the target glycosyltransferase gene sequence. Methods for design and production of such oligonucleotide probes and for performance of Northern or Southern blotting and microarray procedures are well known to those skilled in the art.

Various methods for modification of oligonucleotides by 3', 5' or sequence modification are known to those skilled in the art including, but not limited to amino-modification, carboxy-modification, thiol-modification, aldehyde-modification, chemical phosphorylation modification, spacer, linker or dendrimer modification and many other methods.

The indicator means may be any indicator means known to those skilled in the art including but not limited to a chromogenic, radiolabel or fluorescent indicator molecule. Typically, the indicator means is incorporated into the cDNA or mRNA of step (a) during the amplification step.

Many well-known methods and kits for labelling of cDNA or mRNA during amplification are known to those skilled in the art.

We disclose that the kit may also comprise computer readable instructions for any one or more of:
(a) determining the subject sample glycosyltransferase gene expression profile;
(b) identifying cancer type by:
   i. calculating the distance of the gene expression profile of the subject sample to each of a set of glycosyltransferase gene expression centroids from known cancer types; and
   ii. assigning the subject sample to a cancer type wherein the cancer type is determined as that corresponding to the nearest glycosyltransferase gene expression centroid compared with the glycosyltransferase gene expression profile from the subject sample; and
(c) classifying and/or identifying cancer subtype by:
   i. performing unsupervised consensus clustering analysis of the glycosyltransferase gene expression profile from the subject having known cancer type compared with glycosyltransferase gene expression profiles from a set of reference or training glycosyltransferase genes from subtypes of the corresponding cancer to that of the subject;
   ii. calculating the distance of the gene expression profile of the subject sample to each of a set of glycosyltransferase gene expression centroids from subtypes of the corresponding cancer to that of the subject; and
   iii. assigning the subject sample to a cancer subtype wherein the cancer subtype is determined as that corresponding to the nearest glycosyltransferase gene expression centroid compared with the glycosyltransferase gene expression profile from the subject sample.

Survival information may be known for the reference or training set of glycosyltransferase gene expression profiles or for the glycosyltransferase gene expression centroids from subtypes of the corresponding cancer to that of the subject, in which case the method may optionally include computer readable instructions for identifying the survival prognosis of the subject based on the survival prognosis of the subtype cluster within which the test glycosyltransferase gene expression profile groups.

According to a further aspect of the invention there is provided the use of the method of the invention in the selection of a treatment regime for a subject suspected of having, or known to have cancer comprising performing the method of the invention on a sample from the subject and then selection of the appropriate treatment regime for the cancer type or subtype identified in the subject.

The method of the invention may be performed prior to a subject undergoing therapeutic treatment for cancer and then again subsequent to the subject having undergone therapeutic treatment for cancer, wherein a difference in the glycosyltransferase gene expression levels from before treatment to after treatment is indicative of the efficacy of the therapeutic treatment.

According to a further aspect of the invention there is provided a method of selection of a treatment regime for a subject suspected of having, or known to have cancer comprising performing the method of the invention on a sample from the subject and then selection of the appropriate treatment regime for the cancer type or subtype identified in the subject.

The method of selection may be performed prior to a subject undergoing therapeutic treatment for cancer and then again subsequent to the subject having undergone therapeutic treatment for cancer, wherein a difference in the glycosyltransferase gene expression levels from before treatment to after treatment is indicative of the efficacy of the therapeutic treatment.

We disclose further a method of treatment of a subject suspected of having, or known to have cancer, the method comprising:
(a) performing the method of the invention on a sample from the subject;
(b) identifying the subject's cancer type and optionally the subject's cancer subtype by the method of the invention;
(c) based on the results of step (b), determining whether to initiate treatment, modify the treatment regimen, or discontinue treatment of the subject; and
(d) optionally, initiating treatment, modifying the treatment regimen or discontinuing treatment.

The method of treatment may be performed prior to the subject undergoing therapeutic treatment for cancer and then again subsequent to the subject having undergone therapeutic treatment for cancer, wherein a difference in the glycosyltransferase gene expression profile of the subject from before treatment to after treatment is indicative of the efficacy of the therapeutic treatment.

We disclose further a kit according to the invention for use in a method of treating cancer or selection of a treatment regime for a subject comprising the steps of:
(a) performing the method of the invention on a sample from the subject;
(b) identifying the subject's cancer type and optionally classification of the subject's cancer subtype by the method of the invention;
(c) based on the results of step (b), determining whether to initiate treatment, modify the treatment regimen, or discontinue treatment of the subject; and
(d) optionally, initiating treatment, modifying the treatment regimen or discontinuing treatment.

We disclose further glycosyltransferase gene polynucleotide sequences selected from the group consisting of the glycosyltransferase genes listed in Table 1 or fragments thereof for use with the method of the invention.

### BRIEF DESCRIPTION OF THE DRAWINGS

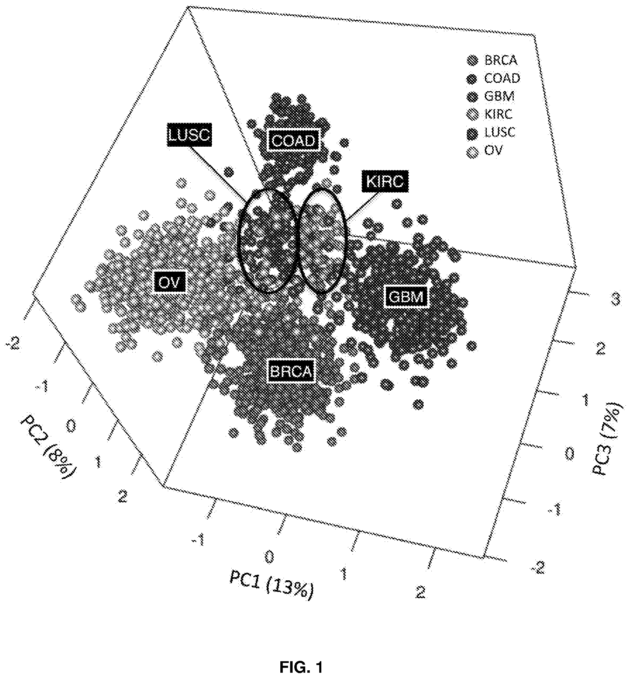

- **Figure 1**: shows the segregation of six TCGA cancer types using Principal Component Analysis (PCA) of the expression profile of 210 glycosyltransferase genes. Biplots (A and B) show separation of six investigated cancer types (i.e. breast: breast invasive carcinoma [BRCA, n=531], ovarian: ovarian serous cystadenocarcinoma [OV, n=578], colon: colon adenocarcinoma [COAD, n=154], lung: lung squamous cell carcinoma [LUSC, n=155], brain: glioblastoma multiforme [GBM, n=403] and kidney: kidney renal clear cell carcinoma [KIRC, n=72]). The numbers in the parenthesis illustrate the amount of variation in the dataset explained by PC1, PC2 and PC3. Circles are shown centeral 90% of the samples for each cancer type;

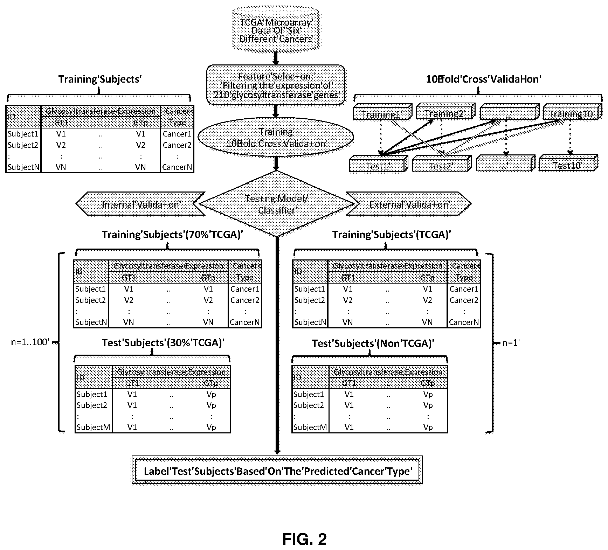

- **Figure 2**: shows the flow chart of supervised classification/class identification of six TCGA cancer types along with the validation steps. The performance of the PAM classifier was examined using 10-fold cross validation, internal and external tests. For the purpose of classifier error estimation in the assignment of samples to the right cancer types, a 10-fold cross validation was carried out. Furthermore, for the purpose of internal validation, the glycosyltranseferases' expression dataset was randomly split hundred times into training (70%) and test (30%) sets. Training sets were used to build a model/classifier, which were then applied to the testing sets. Finally, the median values were used to assign each sample to a specific cancer type. Since training algorithms look for patterns in the training dataset, a classifier that relies on these spurious patterns will have higher accuracy on the training examples than it will on the whole population. Therefore, it is absolutely vital to evaluate the performance of a classifier on an independent test set. For this purpose, TCGA data has been used as training set for an external (independent) test that examine 293 breast cancer samples existing in GPL1390 platform of GSE20624 (Anders *et a*/*.,* 2011). GSE20624 (GPL1390) data is not included in TCGA while it uses the same microarray platform with TCGA datasets, however, only 177 glycosyltransferase are common between training (TCGA based) and this dataset;

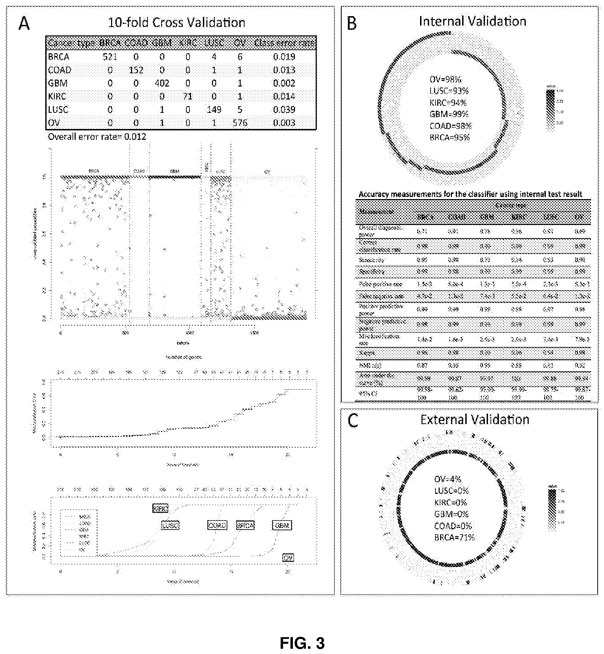

- **Figure 3**: shows the evaluation of the PAM model/classifier made by the expression profile of 210 glycosyltransferase genes in six TCGA cancer types. A) 10-fold Cross Validation (CV). Table summarize the result of 10-fold cross validation, which estimate accuracy of the model/classifier when an independent data set is used for tumour type identification. B) Threshold plots were used to decide on the number of genes in training set, which give the less error rate. In this study the threshold was set to zero (t=0), which produces the least overall error rate in 10-fold cross validation and also to prevent the bias result of internal validation. C) Internal Validation. The heatmap illustrates the result of 100 internal tests. Percentage values on the heatmap columns show the fraction of samples, which assign to the right tumour type with posterior probability ≥ 0.95. D) External Validation. The heatmap illustrates the result of External test, which evaluates the performance of the model/classifier on an independent test set (GSE20624, non-TCGA). TCGA data set has been used as training for an external (independent) test that examine 293 breast cancer samples existing in GPL1390 platform of GSE20624 (Anders *et al.,* 2011). GSE20624 (GPL1390) data is not included in TCGA while it uses the same microarray platform with TCGA datasets, however, only 177 glycosyltransferase are common between training (TCGA based) and this dataset. Percentage values on the heatmap columns show the fraction of samples, which assign to each tumour type with posterior probability ≥ 0.95;

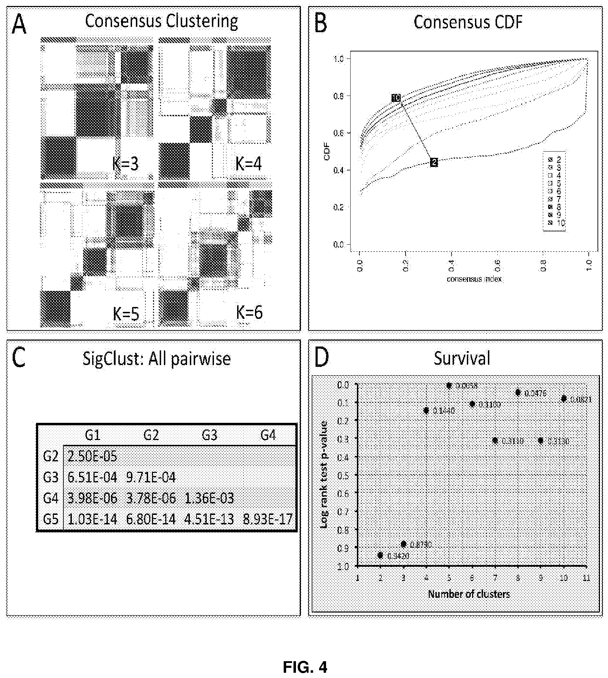

- **Figure 4**: shows the class discovery of five breast cancer subtypes by gene expression profiling of 210 glycosyltransferase genes. (A) Consensus clustering matrix of 467 breast cancer (breast invasive carcinoma) samples with survival and PAM50 subtyping information in TCGA for k=3 to k=6 using k-means clustering method. (B) Delta area for k=2 to k=10, which shows relative change in areae under Consensus Cumulative Distribution Function (CDF) curve. (C) SigClust p-values for all pairwise comparisons of five clusters/groups. (D) Log rank test p-values investigating the relation of number of subtypes (k=2 to k=10) with survival information. G1: group 1 (n=97), G2: group 2 (n=91), G3: group 3 (n=93), G4: group 4 (n=103), G5: group 5 (n=83);

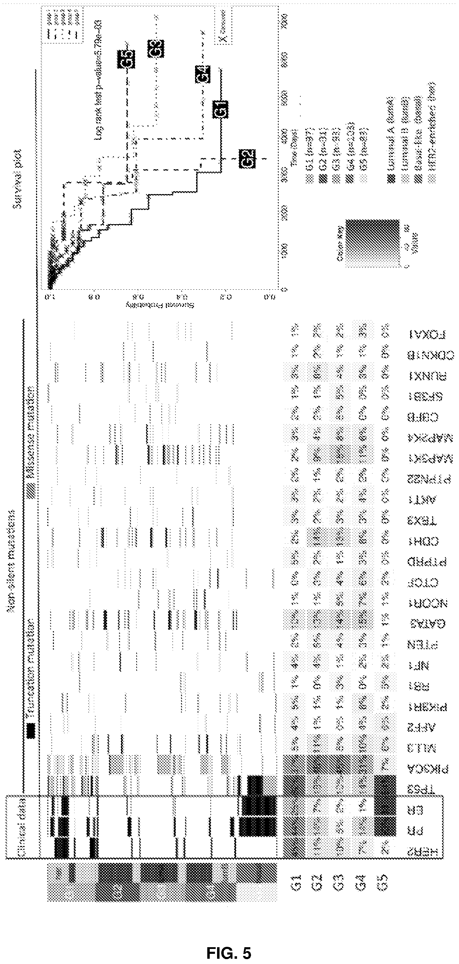

- **Figure 5**: shows the result of survival analysis of TCGA breast cancer samples with respect to the identified subtypes. The survival curves comparing five breast cancer mRNA-expression subtypes (group 1 - group 5) discovered by profiling the expression of 210 glycosyltransferase genes; group 1 (n=97), group 2 (n=91), group 3 (n=93), group 4 (n=103) and group 5 (n=83).

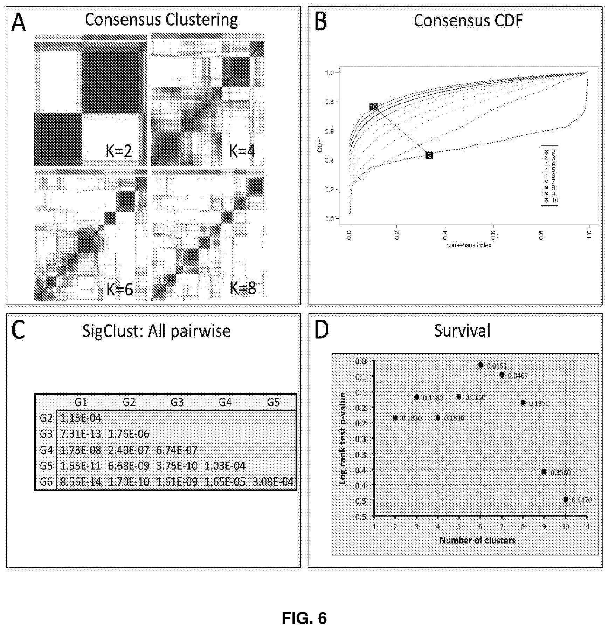

- **Figure 6**: shows the clinical data and somatic mutation patterns comparing five breast cancer subtypes (Group 1 - Group 5) discovered by profiling the expression of 210 glycosyltransferase genes. Tumour samples with clinical data and somatic mutation information (n= 467) in TCGA Agilent Microarray dataset for breast (breast invasive carcinoma) are grouped in five subtypes (Group 1 - Group 5, illustrated with a separate side bar, GT) based on their glycosyltransferase expression: Group 1 (n=97), Group 2 (n=91), Group 3 (n=93), Group 4 (n=103) and Group 5 (n=83) (sample grouping based on the TCGA 2012 study is illustrated with a separate side bar, PAM50). From left to right columns show clinical data and mutation frequencies in heatmap (top) and percentage (bottom) within samples for HER2, PR and ER (positive: white, negative: black). Somatic mutation patterns and frequencies for significantly mutated genes (wild type: white, mutatated: black);

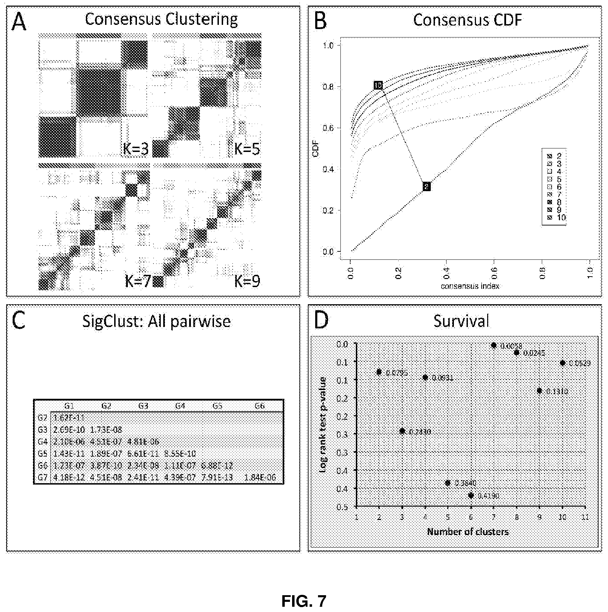

- **Figure 7**: shows the class discovery of six ovarian cancer subtypes by gene expression profiling of 210 glycosyltransferase genes. (A) Consensus clustering matrix of 529 ovarian cancer (ovarian serous cystadenocarcinoma) samples with survival information in TCGA for k=2 to k=8 using k-means clustering method. (B) Delta area for k=2 to k=10, which shows relative change in areae under Consensus Cumulative Distribution Function (CDF) curve. (C) SigClust p-values for all pairwise comparisons of six clusters/groups. (D) Log rank test p-values investigating the relation of number of subtypes (k=2 to k=10) with survival information. G1: group 1 (n=59), G2: group 2 (n=97), G3: group 3 (n=107), G4: group 4 (n=78), G5: group 5 (n=79), G6: group 6 (n=109);

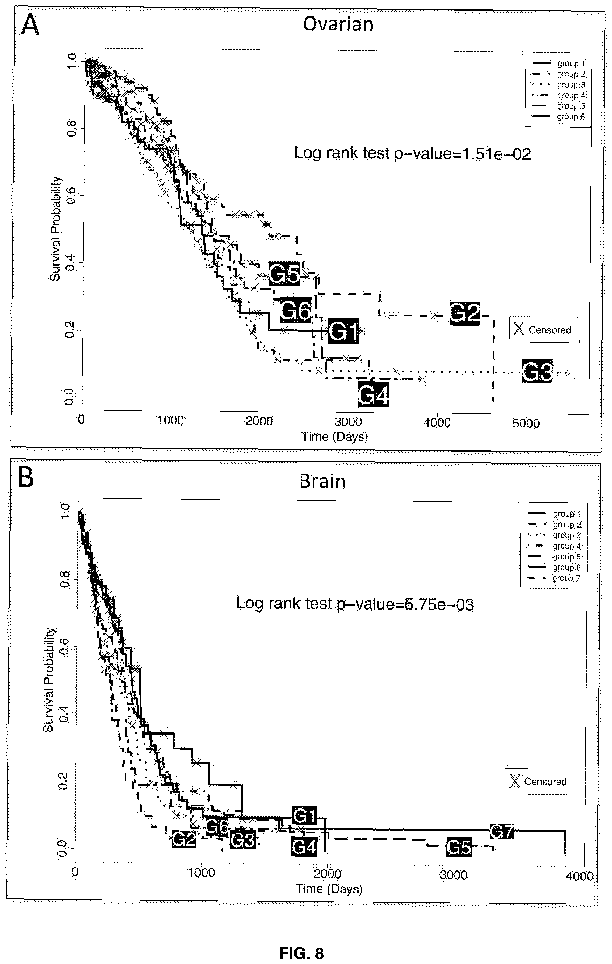

- **Figure 8**: shows the survival analysis of ovarian cancer with respect to the identified subtypes using gene expression profile of 210 glycosyltransferase genes. The plot illustrates Kaplan-Meier survival plot and p-value of the log rank test analysis according to six subtypes for ovarian cancer (high-grade serous ovarian carcinoma): group 1 (n=59), group 2 (n=97), group 3 (n=107), group 4 (n=78), group 5 (n=79), group 6 (n=109);
- **Figure 9**: shows the class discovery of seven brain cancer subtypes by gene expression profiling of 210 glycosyltransferase genes. (A) Consensus clustering matrix of 399 brain cancer (glioblastoma multiforme) samples with survival information in TCGA for k=3 to k=9 using k-means clustering method. (B) Delta area for k=2 to k=10, which shows relative change in areae under Consensus Cumulative Distribution Function (CDF) curve. (C) SigClust p-values for all pairwise comparisons of seven clusters/groups. (D) Log rank test p-values investigating the relation of number of subtypes (k=2 to k=10) with survival information. G1: group 1 (n=48), G2: group 2 (n=33), G3: group 3 (n=73), G4: group 4 (n=33), G5: group 5 (n=93), G6: group 6 (n=34), G7: group 7 (n=85);
- **Figure 10**: shows the survival analysis of brain cancer with respect to the identified subtypes using gene expression profile of 210 glycosyltransferase genes. The plot illustrates Kaplan-Meier survival plot and p-value of the log rank test analysis according to seven subtypes for brain cancer (glioblastoma multiforme): group 1 (n=48), group 2 (n=33), group 3 (n=73), group 4 (n=33), group 5 (n=93), group 6 (n=34), group 7 (n=85);

### DETAILED DESCRIPTION OF THE INVENTION

This invention relates to a method of identification of cancer types in a subject comprising the use of supervised algorithms that have been trained to evaluate quantified glycosyltransferase gene expression in a sample from the subject. In addition, the method relates to a method of cancer subtype classification and/or identification of a cancer subtype within a specific cancer type comprising the use of unsupervised and supervised algorithms that have been trained to evaluate quantified glycosyltransferase gene expression. The invention further relates to a kit comprising glycosyltransferase gene capture probes or primers for use with the method, and a method of treatment of cancer comprising the use of the method or kit of the invention to select an appropriate treatment regime for a subject and/or to monitor the response of the subject to the cancer treatment.

The term "cancer" has come to describe complex malignant diseases that may not share the same causative agents, aetiology or molecular profiles. The complex nature of a solid tumour approaches that of normal tissues and has been compared to that of an organ with the discovery of the family of cell types making up its microenvironment. In an update to the well cited hallmarks of cancer Hanahan and Weinberg summed up the current understanding of the different roles tumour cells play in the progression of the disease (Hanahan and Weinberg, 2011b). Tumour cells have an altered cellular biochemistry that facilitates evasion of growth suppressors and apoptosis as well as acquire invasive, angiogenic and metastatic properties. More pointedly it is unclear to what extent the degree of specific glycosylation mechanisms interface with the pathways. Neoplastic cells must first invade surrounding cells before they can metastasize. Cancer related oligosaccharide changes have been implicated with the invasive properties of cancer cells (Dall'Olio, 1996b). The structure of complex oligosaccharides are altered on normal cells that are on a neoplastic transformation path to malignancy has been documented over the last few decades. These post translational modifications (PTMs), orchestrated by the regulation of glycosyltransferase genes and the subsequent biochemical action of a glycosyltransferases, play key roles in the progression toward malignancy that is reliant on tumorigenesis, tumour progression and metastasis (Ohtsubo and Marth, 2006; Wang et al., 2009; Li et al., 2010; Li et al., 2013; Liu et al., 2013).

The inventors believe that the glycosyltransferase engineered changes of glycan structures covalently coupled to peptides, proteins and lipids are strongly implicated as signatures in malignant tumour typing and possibly subtyping. The inventors have therefore gone on to assess whether or not by monitoring the expression of glycosyltransferase genes on a genomic scale that this subset of genes provides an accurate means of cancer segregation and further identification of cancer type. Surprisingly, the inventors have shown that using the expression profiles of glycosyltransferase genes not only can different cancer types be identified from an unknown test sample, but furthermore, it is possible to classify cancer subtypes within a specific cancer type and optionally to predict a likely outcome for the subject having a particular cancer subtype.

As used herein, the term "gene" refers to a unit of inheritance, including the protein coding and noncoding transcribed regions, upstream and downstream regulatory regions, transcribed regions, and all variants of the mature transcript.

As used herein the term "cDNA" means synthetic DNA synthesized from a messenger RNA (mRNA) template in a reaction catalysed by a reverse transcriptase enzyme.

As used herein, the terms "mRNA" and "mRNA transcript" are used interchangeably and mean an RNA molecule transcribed from the DNA of a gene.

As used herein, the phrase "clustering algorithm" means a bioinformatic mathematical process or set of rules to be followed in calculations or other problem-solving operations, especially by a computer for partitioning a given data set into groups based on specified features so that the data points within a group are more similar to each other than the points in different groups.

"Centroid" as used herein means the average gene expression for each gene in each cancer class (i.e. each cancer type), divided by the within-class standard deviation for that gene.

"Reference glycosyltransferase gene expression centroids" as used herein means a standardized glycosyltransferase gene expression centroid that has been computed for each cancer type by (i) calculating the average gene expression value for each glycosyltransferase gene selected from the group consisting of the glycosyltransferase genes listed in Table 1 in each cancer type, and (ii) dividing the average gene expression value by the within-class standard deviation for that gene.

"Nearest centroid classification" means (i) obtaining the glycosyltransferase gene expression profile of a test sample, (ii) comparing it to each reference glycosyltransferase gene expression centroid, and (iii) identifying the cancer type of the test sample as the cancer type of the centroid that the test sample is closest to, in squared distance.

"Consensus clustering" means a method of providing quantitative evidence for determining the number and membership of possible clusters within a dataset. The Consensus Clustering method involves subsampling from a set of items, such as microarrays, and determines clusterings of specied cluster counts (k). Then, pairwise consensus values, the proportion that two items occupied the same cluster out of the number of times they occurred in the same subsample, are calculated and stored in a symmetrical consensus matrix for each k (Monti et al, 2006).

As used herein, the term "oligonucleotide" means a short single-stranded nucleic-acid chain (either as an oligodeoxynucleotide or oligoribonucleotide). As used herein the term "polynucleotide" means a linear polymer whose molecule is composed of many nucleotide units.

As used herein, the term "cancer type" means the type of cancer as determined by the type of tissue in which the cancer originates (histological type) or by primary site, or the location in the body where the cancer first developed or the kind of cell from which it is derived, as well the appearance of the cancer cells.

As used herein, the term "cancer subtype" means a secondary classification of the cancer type, falling within the cancer type. It may be referred to as a molecular classification of cancer. In particular, the cancer subtype may be associated with molecular alterations, cancer survival, distinct clinical and pathological characteristics, specific gene expression signatures, and deregulated signalling pathways.

Glycans that mediate crucial pathophysiological events taking place at various stages of tumour progression are considered to be one of the key drivers of cancer biology. The applicants have assessed whether, since changes in specific glycan structures are essential for tumourigenesis, tumour progression and metastasis, identifying differences in the expression patterns of their glycosyltransferase genes might be a descriptive approach for understanding cancer complexity. Glycosyltransferase genes regulate the tissue-specific biosynthesis of glycan structures resulting in structural variations of the glycans.

Interestingly, the applicants have determined that the glycosyltransferase gene expression profile indeed can be used not only to identify the specific cancer types of a test or blind sample, but can also be used to classify or identify the test cancer into a subtype, including linking the subtype a particular survival prognosis.

The molecular techniques referenced herein, including RNA extraction, purification amplification, cDNA amplification, mRNA and cDNA labelling, primers and probes design, Northern and Southern blotting, microarray printing and methods, RT-PCR, RT-qPCR, and real time RT-PCR are all standard methods known to those skilled in the art. Many reference sources are available, including but not limited to: http://www.qiagen.com/resources/molecular-biology-methods/, Green and Sambrook, 2012, and others known to those skilled in the art.

The invention will be described by way of the following examples which are not to be construed as limiting in any way the scope of the invention.

### EXAMPLE 1: SIX CANCERS REPRESENTED IN THE CANCER GENOME ATLAS (TCGA)

### 1. Methods

For the purpose of this study, Agilent Microarray (Agilent 244K custom gene expression) data of 1893 samples from published TCGA (http://tcga-data.nci.nih.gov/tcga/) representing six cancer types including breast: breast invasive carcinoma [BRCA, n=531], ovary: ovarian serous cystadenocarcinoma [OV, n=578], brain: glioblastoma multiforme [GBM, n=403], kidney: kidney renal clear cell carcinoma [KIRC, n=72], colon: colon adenocarcinoma [COAD, n=154] and lung: lung squamous cell carcinoma [LUSC, n=155]) were combined, while normal and control samples were excluded.

A list of human glycosyltransferase genes was retrieved through filtering several publicly available databases such as Kyoto Encyclopedia of Genes and Genomes database (KEGG/GENES) (http://www.genome.jp/kegg/genes.html), Carbohydrate-Active Enzymes database (CAZy) databases (http://www.cazy.org/), and literature search. KEGG/GENES is a pool of manually curated genes retrieved mainly from NCBI RefSeq (Pruitt *et al.,* 2005; Kanehisa *et al.,* 2006; Pruitt *et al.,* 2007). Furthermore, CAZy provides an online and regularly updated access to family classification of CAZymes corresponding to proteins released in the daily releases of GenBank (ftp://ftp.ncbi.nih.gov/genbank/daily-nc) (Lombard *et al.,* 2014). Table 1 contains a list of 210 glycosyltransferase gene symbols and their Entrez number.

The expression dataset of glycosyltransferases was built through combining the TCGA expression datasets of six investigated cancer types (i.e. breast, brain, colon, kidney, lung and ovarian) and further retrieving the expression of 210 glycosyltransferase genes from the combined dataset. The result of batch effect analysis clearly illustrated that no significant batch effects in the dataset (data not shown).

To illustrate the segregation of cancer types based on the expression of glycosyltransferase genes, principal component analysis (see Figure 1) was performed using 'psych' package (Revelle, 2014) in R.

To evaluate the potential of glycosyltransferase expression profile in identifying cancer types, a model/classifier was build using 'pamr' package (Hastie *et al.,* 2003) in R and further evaluate using 10-fold cross validation, internal and external tests (see Figure 2 and 3). A two-by-two confusion matrix (also called a contingency table) was constructed representing the dispositions of the set of samples, see Fawcett (2006) for more information and equations. Furthermore, the ROC (Receiver Operating Characteristics) curve and the area under the ROC (AUC) (Bradley, 1997) were also investigated to evaluate the performance of the classifier using 'pROC' package (Robin *et al.,* 2011) in R (see table 2). Furthermore, the performance of the classifier was evaluated using an independent test set. For this purpose, TCGA data set has been used as training set for an external (independent) test that examine 293 breast cancer samples existing in GPL1390 platform of GSE20624 (Anders *et al.,* 2011) (see Figure 3D).

### 2. Results and Discussion

The microarray expression data of 1893 samples were retrieved from The Cancer Genome Atlas (TCGA) project (http://tcga-data.nci.nih.gov/tcga/), representing six cancer types including breast: breast invasive carcinoma [BRCA, n=531], ovary: ovarian serous cystadenocarcinoma [OV, n=578], brain: glioblastoma multiforme [GBM, n=403], kidney: kidney renal clear cell carcinoma [KIRC, n=72], colon: colon adenocarcinoma [COAD, n=154] and lung: lung squamous cell carcinoma [LUSC, n=155]). The applicant further combined and filtered the data to derive at the expression of 210 glycosyltransferase genes.

**Table 1: List of 210 glycosyltransferase genes present in TCGA Agilent microarray expression dataset.**

| Gene Symbol | Entrez ID. | Gene Symbol | Entrez ID. | Gene Symbol | Entrez ID. |
|---|---|---|---|---|---|
| A3GALT2 | 127550 | FUT11 | 170384 | MGAT3 | 4248 |
| A4GALT | 53947 | FUT2 | 2524 | MGAT4A | 11320 |
| A4GNT | 51146 | FUT3 | 2525 | MGAT4B | 11282 |
| ABO | 28 | FUT4 | 2526 | MGAT5 | 4249 |
| ALG10 | 84920 | FUT5 | 2527 | MGAT5B | 146664 |
| ALG11 | 440138 | FUT6 | 2528 | NAGA | 4668 |
| ALG12 | 79087 | FUT7 | 2529 | NEU1 | 4758 |
| ALG13 | 55849 | FUT8 | 2530 | NEU2 | 4759 |
| ALG14 | 199857 | FUT9 | 10690 | NEU3 | 10825 |
| ALG2 | 85365 | GAL | 51083 | NEU4 | 129807 |
| ALG3 | 10195 | GALC | 2581 | OGT | 8473 |
| ALG5 | 29880 | GALNT10 | 55568 | PIGA | 5277 |
| ALG6 | 29929 | GALNT11 | 63917 | PIGB | 9488 |
| ALG8 | 79053 | GALNT12 | 79695 | PIGC | 5279 |
| ALG9 | 79796 | GALNT13 | 114805 | PIGH | 5283 |
| B3GALNT1 | 8706 | GALNT14 | 79623 | PIGM | 93183 |
| B3GALNT2 | 148789 | GALNT2 | 2590 | PIGP | 51227 |
| B3GALT1 | 8708 | GALNT3 | 2591 | PIGQ | 9091 |
| B3GALT2 | 8707 | GALNT4 | 8693 | PIGV | 55650 |
| B3GALT4 | 8705 | GALNT5 | 11227 | PIGX | 54965 |
| B3GALT5 | 10317 | GALNT6 | 11226 | PIGY | 84992 |
| B3GALT6 | 126792 | GALNT7 | 51809 | PIGZ | 80235 |
| B3GALTL | 145173 | GALNT8 | 26290 | PLOD3 | 8985 |
| B3GAT1 | 27087 | GALNT9 | 50614 | POFUT1 | 23509 |
| B3GAT3 | 26229 | GALNTL1 | 57452 | POFUT2 | 23275 |
| B3GNT1 | 11041 | GALNTL2 | 117248 | POMGNT1 | 55624 |
| B3GNT2 | 10678 | GALNTL4 | 374378 | POMT1 | 10585 |
| B3GNT3 | 10331 | GALNTL5 | 168391 | POMT2 | 29954 |
| B3GNT4 | 79369 | GALT | 2592 | PYGM | 5837 |
| B3GNT5 | 84002 | GBGT1 | 26301 | RFNG | 5986 |
| B3GNT6 | 192134 | GCNT1 | 2650 | RPN1 | 6184 |
| B3GNT7 | 93010 | GCNT2 | 2651 | RPN2 | 6185 |
| B3GNT8 | 374907 | GCNT3 | 9245 | ST3GAL1 | 6482 |
| B3GNTL1 | 146712 | GCNT4 | 51301 | ST3GAL2 | 6483 |
| B4GALNT1 | 2583 | GGTA1 | 2681 | ST3GAL3 | 6487 |
| B4GALNT2 | 124872 | GLA | 2717 | ST3GAL4 | 6484 |
| B4GALNT3 | 283358 | GLB1 | 2720 | ST3GAL5 | 8869 |
| B4GALNT4 | 338707 | GLT1D1 | 144423 | ST3GAL6 | 10402 |
| B4GALT2 | 8704 | GLT25D1 | 79709 | ST6GAL1 | 6480 |
| B4GALT3 | 8703 | GLT25D2 | 23127 | ST6GAL2 | 84620 |
| B4GALT4 | 8702 | GLT6D1 | 360203 | ST6GALNAC1 | 55808 |
| B4GALT5 | 9334 | GLT8D1 | 55830 | ST6GALNAC2 | 10610 |
| B4GALT6 | 9331 | GLT8D2 | 83468 | ST6GALNAC3 | 256435 |
| B4GALT7 | 11285 | GLT8D3 | 283464 | ST6GALNAC4 | 27090 |
| C1GALT1 | 56913 | GLT8D4/GXYLT2 | 727936 | ST6GALNAC5 | 81849 |
| C1GALT1C1 | 29071 | GTDC1 | 79712 | ST6GALNAC6 | 30815 |
| CEECAM1 | 51148 | GYG1 | 2992 | ST8SIA1 | 6489 |
| CHGN/GALNACT1 | 55790 | GYG2 | 8908 | ST8SIA2 | 8128 |
| CHPF | 79586 | GYLTL1B | 120071 | ST8SIA3 | 51046 |
| CHSY.2 | 337876 | GYS1 | 2997 | ST8SIA4 | 7903 |
| CHSY1 | 22856 | GYS2 | 2998 | ST8SIA5 | 29906 |
| CSGLCA.T | 54480 | HAS1 | 3036 | STT3A | 3703 |
| DAD1 | 1603 | HAS2 | 3037 | STT3B | 201595 |
| DDOST | 1650 | HAS3 | 3038 | UGCG | 7357 |
| DPAGT1 | 1798 | KDELC1 | 79070 | UGT1A6 | 54578 |
| DPM1 | 8813 | KDELC2 | 143888 | UGT1A8 | 54576 |
| DPM2 | 8818 | KTELC1 | 56983 | UGT2A1 | 10941 |
| DPM3 | 54344 | LARGE | 9215 | UGT2B10 | 7365 |
| EIF2B3 | 8891 | LFNG | 3955 | UGT2B11 | 10720 |
| EIF2B5 | 8893 | LOC152586 | 152586 | UGT2B15 | 7366 |
| EXT1 | 2131 | MAN1A1 | 4121 | UGT2B17 | 7367 |
| EXT2 | 2132 | MAN1A2 | 10905 | UGT2B28 | 54490 |
| EXTL1 | 2134 | MAN1B1 | 11253 | UGT2B4 | 7363 |
| EXTL2 | 2135 | MAN1C1 | 57134 | UGT2B7 | 7364 |
| EXTL3 | 2137 | MAN2A1 | 4124 | UGT3A1 | 133688 |
| FLJ21865 | 64772 | MAN2A2 | 4122 | UGT3A2 | 167127 |
| FUCA1 | 2517 | MANBA | 4126 | UGT8 | 7368 |
| FUCA2 | 2519 | MFNG | 4242 | WBSCR17 | 64409 |
| FUT1 | 2523 | MGAT1 | 4245 | XYLT1 | 64131 |
| FUT10 | 84750 | MGAT2 | 4247 | XYLT2 | 64132 |

To assess the ability of glycosyltransferase expression profile in separating different cancer types Principal Component Analysis (PCA) (Hotelling, 1933) was performed in which the first three principal components with the highest variances (PC1, PC2 and PC3) explained 28% of variations between the cancer types assessed and were clearly shown to separate different cancer types (see Figure 1).

To evaluate the potential of glycosyltransferase expression profile in identifying cancer types, a model/classifier was build using 'pamr' package and for the purpose of error estimation in the assignment of samples to the right cancer types, a 10-fold cross validation technique was carried out using 'pamr' package. In addition, internal and independent/external tests were carried out to evaluate the performance of the pam classifier using the expression of glycosyltransferase genes (see Figure 2).

For the purpose of internal validation, the glycosyltranseferases' expression dataset was randomly split hundred times into training (70%) and test (30%) sets. Training sets were used to build the models/classifiers, which were then applied to the testing sets. Finally, the median values were used to assign each sample to a specific cancer type (see Figure 2). The result of this analysis (see Figure 3C) was used for accuracy measurement calculation summarized in table 2. The accuracy measures derived from a confusion matrix, the area under the receiver operating characteristic (ROC) curve and its confidence interval (CI) for internal test, clearly shows the potential of gene expression profiling of glycosyltransferase in tumour type prediction/identification with high accuracy (> 0.98), sensitivity (> 0.93) and specificity (> 0.98) for all investigated cancer types (see table 2), while at least 93% of test samples are assigned to the right cancer type in internal test (see Figure 3C).

**Table 2: Accuracy measurements for the classifier using the result of internal test. The accuracy measures derived from a confusion matrix, the area under the receiver operating characteristic (ROC) curve and its confidence interval (CI) for internal test.**

| **Measurement** | **Cancer type** | | | | | |
|---|---|---|---|---|---|---|
| | **BRCA** | **COAD** | **GBM** | **KIRC** | **LUSC** | **OV** |
| Overall diagnostic power | 0.71 | 0.91 | 0.78 | 0.96 | 0.91 | 0.69 |
| Correct classification rate | 0.98 | 0.99 | 0.99 | 0.99 | 0.99 | 0.99 |
| Sensitivity | 0.95 | 0.98 | 0.99 | 0.94 | 0.93 | 0.98 |
| Specificity | 0.99 | 0.98 | 0.99 | 0.99 | 0.99 | 0.99 |
| False positive rate | 1.5e-3 | 6.0e-4 | 1.3e-3 | 5.0e-4 | 2.3e-3 | 5.3e-3 |
| False negative rate | 4.7e-2 | 1.3e-2 | 7.4e-3 | 5.5e-2 | 6.4e-2 | 1.3e-2 |
| Positive predictive power | 0.99 | 0.99 | 0.99 | 0.98 | 0.97 | 0.98 |
| Negative predictive power | 0.98 | 0.99 | 0.99 | 0.99 | 0.99 | 0.99 |
| Misclassification rate | 1.4e-2 | 1.6e-3 | 2.6e-3 | 2.6e-3 | 7.4e-3 | 7.9e-3 |
| Kappa | 0.96 | 0.98 | 0.99 | 0.96 | 0.94 | 0.98 |
| NMI n(s) | 0.87 | 0.95 | 0.96 | 0.88 | 0.85 | 0.92 |
| Area under the curve (%) | 99.99 | 99.87 | 99.97 | 100 | 99.88 | 99.94 |
| 95% CI | 99.98-100 | 99.62-100 | 99.90-100 | 99.99-100 | 99.75-100 | 99.87- 100 |

Since training algorithms look for patterns in the training dataset, a classifier that relies on these imitation patterns will have higher accuracy on the training examples than it will on the whole population. Therefore, it is absolutely critical to evaluate the performance of a classifier on an independent test set. For this purpose, training sets of previous test (internal test) have been used for an external (independent) test that examine 293 breast cancer samples existing in GPL1390 platform of GSE20624 (Anders *et al.,* 2011). GSE20624 (GPL1390) data is not included in TCGA while it uses the same microarray platform with TCGA datasets, however, only 177 glycosyltransferase are common between training (TCGA based) and this dataset. The result of this test was illustrated as a heatmap (see Figure 3D).

We thus, determined estimations for tumour type separation of more than 93% and 70% (posterior probability ≥ 0.95) in internal and external tests, respectively (see Figure 3C and D).

### 3. Conclusion

DNA microarray experiments generate large datasets with simultaneous monitoring of thousands of gene expressions. Accurate identification of different tumour types and subtypes from the unknown samples in such high-dimensional data is difficult but often crucial for successful diagnosis and treatment.

The results of this study show that the expression profiles of glycosyltransferase genes are significantly altered between different cancer types and these differences are enough to identify cancer type and optionally subtypes in a subject. Therefore, this study offers a novel and improved alternative method compared to current cancer diagnosis techniques.

### EXAMPLE 2: CANCER SUBTYPING

### 1. Methods

The inventors have assessed the use of glycosyltransferase gene expression profiles in subtype classification and identification. Samples from three different cancers were obtained: breast, ovarian and brain cancer, and these were analysed for the provision of a quantitative evidence for the prediction of a number of possible subtypes within the each cancer dataset by consensus clustering plus class discovery technique (Wilkerson and Hayes, 2010) using 'ConsensusClusterPlus' package (Monti *et al.,* 2003) in R. Delta area, the area under Cumulative Distribution Function (CDF) curve is a graphical representation to illustrate at what number of clusters there is no significant increase in CDF curve. Furthermore, to group samples into subtypes based on the expression of glycosyltransferase genes, a k-means clustering was performed using 'cluster' package (Maechler *et al.,* 2012) in R. In addition, cluster significance was evaluated using 'SigClust' package (Huang *et al.,* 2012) in R. To investigate whether the identified groups (using k-means clustering), specific to each of the cancer types may represent clinically distinct subgroups of patients; univariate survival analyses (comparing subtypes, k=2 to k=10, with respect to the overall survival) was performed using 'survival' package (Therneau, 2013) in R.

In case of breast cancer, previously identified normal-like (TCGA, 2012), metastatic samples and the samples with missing survival information in the corresponding patient were excluded. Furthermore, clinical and somatic mutation patterns along with survival curves were compared within the context of the five discovered subtypes by the expression of glycosyltransferase genes.

In terms of ovarian and brain cancers, the samples with missing survival information in the corresponding patient were excluded.

### 2. Results and Discussion

In addressing the ability of glycosyltransferase genes in each of the cancer types to provide subtype classification, the inventors performed consensus clustering that allows a quantitative and visual assessment of the estimated number of unsupervised subtypes in a particular cancer (Prat *et al.,* 2013a). The results indicate that clustering stability increases from k=2 to k=10 (see Figures 4, 7 and 9) and it may explain the heterogeneity observed in each cancer type, while further identifying molecular subtypes within or in addition to previously identified classes.

### Breast Cancer Subtypes

The inventor's results pertaining to the prognostic evaluation of breast cancer subtypes using the expression profile of 210 glycosyltransferase genes (see Figure 4) indicate that survival curves significantly differ between subtypes when breast cancer samples are divided into five groups (see Figure 4D and 5). According to the study carried out by Perou *et al.* (2000), Sorlie *et al.* (2001) and several others, the existence of four major breast cancer subtypes (Luminal A, Luminal B, Basal-like and HER2-enriched) has become a consensus in breast cancer classification. Furthermore, a study carried out by The Cancer Genome Atlas Network, TCGA (2012), provided key insights into these four previously defined breast cancer subtypes and explains significant molecular heterogeneity in each subtypes.

In line with these findings, the inventor's results show the most significant difference between breast cancer subtypes when data is divided into five groups (see Figure 4D and 5, Log rank test p-value=5.79e-03). Furthermore, clinical and somatic mutation patterns were compared within the context of the five detected subtypes (Group 1 - Group 5) using the expression profile of 210 glycosyltransferase genes (see Figure 6). The result of this analysis was shown that there is a significant overlap between Group 1 and HER2-enriched, and Group 5 and basal-like subtypes, while luminal (A and B) are mainly distributed between other three subtypes (Group 2 - Group 4). Clinical patterns were significantly more conserved within Group 1 (HER2+: 45%, PR-: 44% and ER-: 25%, considered as HER2-enriched) and Group 5 (HER2+: 2%, PR-: 93% and ER-: 84%, considered as basal-like) in compare to other three subtypes (see Figure 6).

The samples in Group 1 and Group 5 subtypes are mostly mutated for TP53 (62% and 84% respectively), while Group 1 and Group 5 are differently mutated for PIK3CA (37% and 7% respectively). TP53 mutations are significantly enriched in HER2+ or ER- tumours, while a high frequency of PIK3CA mutations was reported in HER+ tumours (TCGA, 2012). Therefore, the investigation of mutation frequency of TP53 and PIK3CA in Group 1 (62% and 37% respectively) and Group 5 (84% and 7% respectively) are extra evidence beside the clinical data confirming these two identified subtypes using glycosyltransferase expression profile might be HER2-enriched and basal-like subtypes and they are different from luminal subtypes. Furthermore, GATA3 mutations rarely found in Group 1 (1%) compare to other groups (10%, 13%, 14% and 15% for Group 1 to Group 4 respectively) in this study (see Figure 6). Since GATA3 mutations are only observed in luminal subtypes or ER+ tumours (TCGA, 2012), which might be extra support for Group 5 to be considered as basal-like subtype that glycosyltransferase expression profile clearly identified and separated this subtype from other subtypes.

Among luminal subtypes (Group 2 - Group 4), Group 4 has the lowest rate of HER2+ and ER- (HER2+: 7% and ER-: 1%), while Group 2 and Group 3 have the highest ER- (7%) and lowest PR- (5%) respectively. The samples in Group 4 (considered as luminal/ER+/HER2-) subtype are moderately mutated for TP53 (14%) and PIK3CA (31%), followed by GATA3 (15%), MAP3K1 (11%) and MLL3 (10%) (see Figure 6). Estrogen is the prime growth regulator of ER-positive breast cancer and GATA3 is an ER-regulated gene and play key roles in ER-mediated target gene regulation and it is important for the genomic action of the ER (Ma *et al.,* 2013). Mutations in this gene could potentially mark disease that is sensitive to endocrine therapy (Ellis *et al.,* 2012a).

Furthermore, the rate of GATA3 mutation in luminal subtypes (Group 2: 13%, Group 3: 14% and Group 4: 15%) (see Figure 6) is in line with the result of the other studies, which reported the GATA3 mutation at ∼10% frequency investigating a significant number of luminal tumours (Banerji *et al.,* 2012; Ellis *et al.,* 2012a; Stephens *et al.,* 2012; TCGA, 2012). Another feature of the somatic mutated gene list in ER+/HER2- breast cancer is the presence of additional genes previously implicated in leukemia and myelodysplasia such as MLL3 and RUNX1 (Ellis and Perou, 2013). MLL family members encode histone trimethyltransferases - considered positive global regulators of gene transcription, with functions that include regulation of estrogen receptor gene expression (Won Jeonq *et al.,* 2012). Theoretically MLL mutant tumors, as well as others with mutations in genes that are epigenetic regulators of gene expression, might be sensitive to the burgeoning class of drugs that target post-translational histone modifications (Huang *et al.,* 2011).

Group 2 and Group 3 subtypes are significantly enriched for luminal A mRNA subtype. The samples in Group 3 subtype (considered as luminal/ER+/HER2+) are rarely mutated for TP53 (10%) but mostly mutated for PIK3CA (45%), followed by GATA3 (14%), CDH1 (13%) and MAP3K1 (18%), while the samples in Group 2 subtype (considered as luminal/ER-/HER2+) are mostly mutated for TP53 and PIK3CA (18% and 48% respectively) compare to other luminal subtypes followed by MLL3 (11%), GATA3 (13%) and CDH1 (14%) (see Figure 6). CDH1 mutations are correlated with a significant alteration in HER2 (Ross *et al.,* 2013), while MAP3K1 mutations often coexist with PI3KCA mutations in luminal A and shown to be linked with low proliferative index before and after estrogen deprivation therapy (Ellis *et al.,* 2012a). After TP53, mutation rate of MAP3K1 significantly vary between Group 2 and Group 3 subtypes (9% and 18% respectively) (see Figure 6). MAP3K1 mutation is a high frequency driver mutation for Luminal A disease and therefore also a likely causal molecular event (Ellis and Perou, 2013), which may lead to the molecular subtypes in luminal disease.

In conclusion, investigating the expression profile of glycosyltransferase genes provide a clear distinction between breast cancer subtypes (Luminal A/B, Basal-like and HER2-enriched) and may provide key insights into molecular heterogeneity of luminal subtypes considering subtype-associated gene mutations.

### Ovarian and Brain Cancer Subtypes

To investigate whether the identified groups (using the same methods that have been explained for breast cancer) (see Figures 7 and 9), specific to ovarian and brain cancer types may represent clinically distinct subgroups of patients, the inventor's performed a univariate survival analyses (comparing subtypes with respect to the overall survival). Survival curves were found to significantly differ between different cancer subtypes when ovarian and brain cancers were divided (see Figure 7D, 8, 9D and 10).

In terms of ovarian cancer, the most significant difference between survival curves of subtypes was achieved when data divided into six subtypes (Log rank test p-value=1.51e-02) (see Figure 7D and 8) despite, four, five and six molecular subtypes having been previously reported by The Cancer Genome Atlas Network, TCGA (2011), Tan *et al.* (2013) and Tothill *et al.* (2008), respectively.

Furthermore, the inventors showed the presence of seven subtypes for brain (Log-rank test p-value=5.75e-03) (see Figure 9D and 10), while the number of brain subtypes previously reported by Verhaak *et al.* (2010) and Brennan *et al.* (2013) is four and five, respectively.

### Conclusion

In conclusion, the heterogeneity presented between discovered breast cancer subtypes and homogeneity displayed within each subtype in terms of clinical and mutation patterns may confirm the potential of using the expression profile of glycosyltransferase genes in cancer subtyping and outcome prediction. Furthermore, the significance of the inventor's findings lies not only in finding previously reported cancer subtypes but also their significant correlation with clinical outcome (and mutation patterns in case of breast cancer) provides a supporting evidence for the potential use of glycosyltransferase gene expression profiles in cancer class subtyping.

### 4. Final conclusion

Personalized medicine in the context of cancer, is closely tied with the concept of accurately identifying cancer types and furthermore subtypes of a specific cancer in a subject for accurate diagnosis, predict an outcome and hence advise on better treatment.

Although glycan alteration may be indicative of the specificity of glycan epitopes to their associated cancer group, the fundamental and precise structures of glycans are complicated and largely not understood. Thus, in the absence of this information, the use of glycosyltransferase gene expression profiles is an compelling alternative with the diagnosis potential, with in-depth understanding of the complexity of different cancer types at the translational level.

The altered expression of glycosyltransferase genes whose expressions are significantly related to cancer patient survival represent compelling evidence for the role of these genes in cancer type identification and subtyping. Therefore, glycosyltransferase genes represent useful targets for use in clinical assays, resulting in the development of improved early diagnosis.

### REFERENCES

[1]. D. Hanahan, R. A. Weinberg,Cell 144, (2011a)
[2]. F. Dall'Olio,Clin Mol Pathol. 49, (1996a)
[3]. K. Ohtsubo, J. D. Marth,Cell 126, (2006)
[4]. W. Wang, et al.,Proc Natl Acad Sci U S A 106, (2009)
[5]. M. Li, et al.,J Biosci 35, (2010)
[6]. S. Li, et al.,PLoS One 8, (2013)
[7]. L. Liu, et al.,PLoS One 8, (2013)
[8]. C. K. Anders, et al.,Journal of Clinical Oncology 29, (2011)
[9]. J. T. Leek, et al.,Bioinformatics 28, (2012)
[10]. D. Hanahan, R. A. Weinberg,cell 144, (2011b)
[11]. F. Dall'Olio,Clinical molecular pathology 49, (1996b)
[12]. K. D. Pruitt, et al.,Nucleic Acids Res 33, (2005)
[13]. M. Kanehisa, et al.,Nucleic Acids Res 34, (2006)
[14]. K. D. Pruitt, et al.,Nucleic Acids Res 35, (2007)
[15]. V. Lombard, et al.,Nucleic Acids Res 42, (2014)
[16]. W. Revelle,R package (2014)
[17]. M.R. Green and J. Sambrook, Molecular Cloning: A Laboratory Manual, (2012)
[18]. T. Hastie, et al.,R package (2003)
[19]. T. Fawcett,Pattern Recognition Letters 27, (2006)
[20]. A. P. Bradley,Pattern recognition 30, (1997)
[21]. X. Robin, et al.,BMC bioinformatics 12, (2011)
[22]. H. Hotelling,Journal of educational psychology 24, (1933)
[23]. A. Prat, et al.,Scientific Reports 3, (2013a)
[24]. M. D. Wilkerson, D. N. Hayes,Bioinformatics 26, (2010)
[25]. M. Maechler, et al.,R package (2012)
[26]. S. Monti, et al.,Machine learning 52, (2003)
[27]. H. Huang, et al.,R package (2012)
[28]. T. Therneau,R package (2013)
[29]. TCGA,Nature 490, (2012)
[30]. A. Prat, et al.,Sci Rep 3, (2013b)
[31]. C. M. Perou, et al.,Nature 406, (2000)
[32]. T. Sorlie, et al.,Proc Natl Acad Sci U S A 98, (2001)
[33]. C. X. Ma, et al.,Breast cancer research and treatment 139, (2013)
[34]. M. J. Ellis, et al.,Nature 486, (2012a)
[35]. M. J. Ellis, et al.,Nature 486, (2012b)
[36]. S. Banerji, et al.,Nature 486, (2012)
[37]. P. J. Stephens, et al.,Nature 486, (2012)
[38]. M. J. Ellis, C. M. Perou,Cancer discovery 3, (2013)
[39]. K. Won Jeong, et al.,Molecular Endocrinology 26, (2012)
[40]. Y. Huang, et al.,Breast Cancer Research : BCR 13, (2011)
[41]. J. S. Ross, et al.,Clinical Cancer Research 19, (2013)
[42]. TCGA,Nature 474, (2011)
[43]. T. Z. Tan, et al.,EMBO molecular medicine 5, (2013)
[44]. R. W. Tothill, et al.,Clinical Cancer Research 14, (2008)
[45]. R. Verhaak, et al.,Cancer Cell 17, (2010)
[46]. C. W. Brennan, et al.,Cell 155, (2013)

## Claims

1. An *in vitro* method of identification of cancer type from the group consisting of six cancer types selected from breast invasive carcinoma, lung squamous cell carcinoma, ovarian serous cystadenocarcinoma cancer, glioblastoma multiforme, colon adenocarcinoma, and kidney renal clear cell carcinoma in a test sample from a subject comprising the steps of:
(a) obtaining a glycosyltransferase gene expression profile from a set of 210 glycosyltransferase genes consisting of those listed in Table 1 in the test sample from the subject;
(b) calculating the distance of the gene expression profile of the test sample to a reference set of glycosyltransferase gene expression centroids from the six cancer types; and
(c) assigning the test sample to a cancer type, wherein the cancer type is identified as the cancer type corresponding to the nearest reference set glycosyltransferase gene centroid of the six cancer types glycosyltransferase gene expression centroids compared with the glycosyltransferase gene expression profile from the test sample.

2. The method according to claim 1, comprising the following steps:
(a) obtaining a glycosyltransferase gene expression profile for each of a training set of cancer samples from subjects with each of the six cancer types, wherein the glycosyltransferase genes consist of the 210 glycosyltransferase genes listed in Table 1;
(b) using a supervised algorithm to construct gene expression centroids for each of the six cancer types in the training set;
(c) calculating the distance of the gene expression profile of the test sample to each of glycosyltransferase gene expression centroids from the training set of the six cancer types; and
(d) assigning the test sample to a cancer type wherein the cancer type is identified as that corresponding to the nearest training set glycosyltransferase gene centroid of the six cancer types compared with the glycosyltransferase gene expression profile from the test sample.

3. The method according to either claim 1 or 2, wherein the reference set or training set of cancer types or both consists of cancer types selected from breast, including breast invasive carcinoma, brain, including glioblastoma multiforme, colon, including colon adenocarcinoma, kidney, including kidney renal clear cell carcinoma, lung, including lung squamous cell carcinoma, and ovarian, including ovarian serous cystadenocarcinoma cancer.

4. The method according to any one of the preceding claims, wherein the method further comprises a step of classifying the subtype of the cancer in a test subject having one of the six cancer types, wherein the subtype classification comprises the steps of:
(a) providing a reference set of glycosyltransferase gene expression profiles from subtypes of the corresponding cancer type to that of the subject, wherein the glycosyltransferase genes consist of the 210 glycosyltransferase genes listed in Table 1;
(b) performing unsupervised clustering analysis on the test subject glycosyltransferase gene expression profile and the reference set of glycosyltransferase gene expression profiles; and
(c) classifying the test subject subtype by grouping the test glycosyltransferase gene expression profile into one or more subtypes corresponding to the closest reference glycosyltransferase gene expression profile for a subtype.

5. The method according to any one of the preceding claims, whereby the method further comprises a step of classifying the subtype of the cancer in a test subject having one of the six cancer types, wherein the subtype classification comprises the steps of:
(a) calculating the distance of the gene expression profile of the test sample to each of a set of reference glycosyltransferase gene expression centroids from the subtypes of the corresponding cancer to that of the subject; and
(b) assigning the test sample to a cancer subtype wherein the cancer subtype is identified as that corresponding to the nearest reference set glycosyltransferase gene centroid compared with the glycosyltransferase gene expression profile from the test sample.

6. The method according to any one of claims 2, 3, or 5, wherein the method further comprises a step of classifying the subtype of the cancer in a test subject having one of the six cancer types, wherein the subtype classification comprises the steps of:
(a) using a supervised algorithm to construct gene expression centroids for each of the cancer subtypes in the training set;
(b) calculating the distance of the gene expression profile of the test sample to each of glycosyltransferase gene expression centroids constructed in step (a); and
(c) assigning the test sample to a cancer subtype wherein the cancer subtype is determined as that corresponding to the nearest training set glycosyltransferase gene centroid compared with the glycosyltransferase gene expression profile from the test sample.

7. The method according to any one of claims 4 to 6, wherein survival information for each of the set of reference glycosyltransferase gene expression profiles or centroids is known and the survival prognosis of the test subject is identified based on the survival prognosis of the subtype cluster within which the test glycosyltransferase gene expression profile groups.

8. The method according to any one of claims 4 to 7, wherein the step of subtyping of the cancer after identification of cancer type is performed on samples from subjects identified as having breast, brain or ovarian cancer.

9. The method according to any one of the preceding claims, wherein the step of obtaining a glycosyltransferase gene expression profile further comprises the steps of:
(a) purifying total RNA from the sample of the test subject;
(b) converting the RNA into cDNA by reverse transcription and labelling the cDNA with a fluorescent dye or amplifying mRNA from the RNA and labelling the mRNA with a fluorescent dye;
(c) contacting a microarray slide printed with target oligonucleotide probes specific for the 210 glycosyltransferase genes listed in Table 1 or a gene fragment of each with the fluorescently labelled cDNA or mRNA;
(d) hybridizing the fluorescently labelled cDNA or mRNA to the target oligonucleotide probes; and
(e) quantifying the intensity of fluorescence of cDNA or mRNA bound to their target oligonucleotide probes on the microarray slide, whereby the intensity of fluorescence at each target corresponds to the relative abundance of mRNA transcript for each subject glycosyltransferase gene and is the subject sample glycosyltransferase gene expression profile.

10. The method according to any one of claims 1 to 8, wherein the step of obtaining a glycosyltransferase gene expression profile comprises the steps of:
(a) purifying total RNA from the sample of the test subject;
(b) converting the RNA into cDNA by reverse transcription and labelling the cDNA with a fluorescent dye or a chromogenic substrate, or amplifying mRNA from the RNA and labelling the mRNA with a fluorescent dye or a chromogenic substrate;
(c) contacting a dot blot printed with target oligonucleotide probes specific for the 210 glycosyltransferase genes listed in Table 1 or a gene fragment of each with the labelled cDNA or mRNA;
(d) hybridizing the labelled cDNA or mRNA to the target oligonucleotide probes; and
(e) quantifying the intensity of the hybridized labelled cDNA or mRNA bound to their target oligonucleotide probes on the dot blot, whereby the intensity of the label at each target corresponds to the relative abundance of mRNA transcript for each gene and is the subject sample glycosyltransferase gene expression profile.

11. The method according to any one of claims 1 to 8, wherein the step of obtaining a glycosyltransferase gene expression profile further comprises the steps of:
(a) purifying total RNA from the sample of the test subject;
(b) amplifying cDNA from the RNA by RT-qPCR with specific primers for the 210 glycosyltransferase genes listed in Table 1 or a gene fragment of each;
(c) quantifying the amplified cDNA; and
(d) assessing the relative abundance of the amplified cDNA of each of the genes, whereby the abundance of the amplified cDNA corresponds to the relative abundance of mRNA transcript for each gene and is the subject sample glycosyltransferase gene expression profile.

12. A kit for classifying a cancer type selected from the group consisting of six cancer types selected from breast invasive carcinoma, lung squamous cell carcinoma, and ovarian serous cystadenocarcinoma cancer, glioblastoma multiforme, colon adenocarcinoma, and kidney renal clear cell carcinoma in a test sample from a subject and optionally also cancer subtype in the subject with the use of the method of any one of claims 1 to 11, the kit consisting of
(a) primers for amplification of cDNA or mRNA or
(b) gene specific oligonucleotide probes for hybridizing to cDNA or mRNA in a dot blot procedure; or
(c) gene specific oligonucleotide probes printed on a microarray slide, to which labelled cDNA or RNA can be hybridized in a microarray method and
(d) an indicator means;
wherein the primers or gene specific oligonucleotide probes are specific to the 210 glycosyltransferase genes or fragments thereof listed in Table 1;
(e) optionally, directions for use;
(f) optionally a reference set of glycosyltransferase polynucleotides from the six cancer types or subtypes thereof, wherein the polynucleotides comprise the glycosyltransferase genes listed in Table 1 or fragments thereof; and
(g) optionally computer readable instructions for any one or more of:
A. determining the subject test sample glycosyltransferase gene expression profile;
B. identifying cancer type by:
i. calculating the distance of the gene expression profile of the subject test sample to each of the set of glycosyltransferase gene expression centroids from the six cancer types; and
ii. assigning the subject test sample to the cancer type wherein the cancer type is determined as that corresponding to the nearest glycosyltransferase gene expression centroid compared with the glycosyltransferase gene expression profile from the subject test sample; and
C. classifying and/or identifying the cancer subtype by:
i. performing unsupervised consensus clustering analysis of the glycosyltransferase gene expression profile from the subject test sample having one of the six cancer types compared with the glycosyltransferase gene expression profiles from a set of reference or training glycosyltransferase genes from subtypes of the corresponding cancer to that of the subject;
ii. calculating the distance of the gene expression profile of the subject test sample to each of the set of glycosyltransferase gene expression centroids from subtypes of the corresponding cancer to that of the subject; and
iii. assigning the subject test sample to a cancer subtype wherein the cancer subtype is determined as that corresponding to the nearest glycosyltransferase gene expression centroid compared with the glycosyltransferase gene expression profile from the subject test sample.

13. The kit according to claim 12, wherein survival information for each of the set of reference or training glycosyltransferase gene expression profiles or centroids is known, further comprising computer readable instructions for identification of the survival prognosis of the subject based on the survival prognosis of the subtype cluster within which the test glycosyltransferase gene expression profile groups.

14. A method of selection of a treatment regime for a subject suspected of having, or known to have cancer comprising performing the method of any one of claims 1 to 11 on a sample from the subject and then selection of the appropriate treatment regime for the cancer type or subtype identified in the subject, wherein the method is performed prior to the subject undergoing therapeutic treatment for cancer and then again subsequent to the subject having undergone therapeutic treatment for cancer, wherein a difference in the glycosyltransferase gene expression levels from before treatment to after treatment is indicative of the efficacy of the therapeutic treatment.

## Patentansprüche

1. In-Vitro-Verfahren zur Identifizierung einer Krebsart aus der Gruppe bestehend aus sechs Krebsarten ausgewählt aus invasivem Brustkarzinom, Plattenepithelkarzinom der Lunge, serösem Ovarialzystadenokarzinomkrebs, Glioblastoma multiforme, Dickdarm-Adenokarzinom und Klarzellen-Nierenkarzinom in einer Testprobe eines Patienten, das die folgenden Schritte umfasst:
(a) Erhalten eines Glycosyltransferase-Genexpressionsprofils aus einem Satz von 210 Glycosyltransferase-Genen, die aus jenen bestehen, die in Tabelle 1 aufgelistet sind, in der Testprobe des Patienten;
(b) Berechnen der Entfernung des Genexpressionsprofils der Testprobe zu einem Referenzsatz von Glycosyltransferase-Genexpressionszentroiden aus den sechs Krebsarten; und
(c) Zuweisen der Testprobe zu einer Krebsart, wobei die Krebsart als die Krebsart identifiziert ist, die dem nächsten Referenzsatz-Glycosyltransferase-Genzentroid der Glycosyltransferase-Genexpressionszentroiden der sechs Krebsarten entspricht, im Vergleich mit dem Glycosyltransferase-Genexpressionsprofil aus der letzten Probe.

2. Verfahren nach Anspruch 1, das die folgenden Schritte umfasst:
(a) Erhalten eines Glycosyltransferase-Genexpressionsprofil für jeden eines Trainingssatzes von Krebsproben von Patienten mit jedem der sechs Krebstypen, wobei die Glycosyltransferase-Gene aus den 210 Glycosyltransferase-Genen, die in der Tabelle 1 aufgelistet sind bestehen;
(b) Verwenden eines kontrollierten Algorithmus, um Genexpressions-Zentroide für jeden der sechs Krebsarten in dem Trainingssatz zu konstruieren;
(c) Berechnen der Entfernung des Genexpressionsprofils der Testprobe zu jedem der Glycosyltransferase-Genexpressionszentroide von dem Trainingssatz der sechs Krebsarten; und
(d) Zuweisen der Testproben zu einer Krebsart, wobei die Krebsart als jene identifiziert ist, die dem nächsten Trainingssatz-Glycosyltransferase-Genzentroid der sechs Krebsarten, verglichen mit dem Glycosyltransferase-Genexpressionsprofil aus der Testprobe, entspricht.

3. Verfahren nach Anspruch 1 oder 2, wobei der Referenzsatz oder Trainingssatz von Krebsarten oder beide aus Krebsarten bestehen, die ausgewählt sind aus Brustkrebs, einschließlich invasiven Brustkarzinoms, Hirnkrebs, einschließlich Glioblastoma multiforme, Dickdarmkrebs, einschließlich Dickdarm-Adenokarzinom, Nierenkrebs, einschließlich Klarzellen-Nierenkarzinom, Lungenkrebs, einschließlich Plattenepithelkarzinom der Lunge, und Ovarialkrebs, einschließlich serösen Ovarialzystadenokarzinomkrebs.

4. Verfahren nach einem der vorhergehenden Ansprüche, wobei das Verfahren ferner einen Schritt des Klassifizierens der Unterart des Krebses in einem Testpatienten umfasst, der eine der sechs Krebsarten aufweist, wobei die Unterartenklassifizierung die folgenden Schritte umfasst:
(a) Bereitstellen eines Referenzsatzes von Glycosyltransferase-Genexpressionsprofilen von Unterarten der Krebsart, die jener des Patienten entspricht, wobei die Glycosyltransferase-Gene aus den 210 Glycosyltransferase-Genen bestehen, die in Tabelle 1 aufgelistet sind;
(b) Durchführen von nicht kontrollierter Cluster-Analyse des Glycosyltransferase-Genexpressionsprofils des Patienten und des Referenzsatzes von Glycosyltransferase-Genexpressionsprofilen; und
(c) Klassifizieren der Unterart des Testpatienten durch Gruppieren des Test-Glycosyltransferase-Genexpressionsprofils in eine oder mehrere Unterarten, die dem nächsten Referenz-Glycosyltransferase-Genexpressionsprofil für eine Unterart entsprechen.

5. Verfahren nach einem der vorhergehenden Ansprüche, wobei das Verfahren ferner einen Schritt des Klassifizierens der Unterart des Krebses in einem Testpatienten umfasst, der eine der sechs Krebsarten aufweist, wobei die Klassifizierung der Unterart die folgenden Schritte umfasst:
(a) Berechnen der Entfernung des Genexpressions-Profils der Testprobe zu jedem eines Satzes von Glycosyltransferase-Genexpressionszentroiden von den Unterarten des entsprechenden Krebses zu jenem des Patienten; und
(b) Zuweisen einer Testprobe zu einer Krebsunterart, wobei die Krebsunterart als jene identifiziert ist, die dem nächsten Referenzsatz-Glycosyltransferase-Genzentroid im Vergleich mit dem Glycosyltransferase-Genexpressionsprofil aus der Testprobe entspricht.

6. Verfahren nach einem der Ansprüche 2, 3 oder 5, wobei das Verfahren ferner einen Schritt des Klassifizierens der Unterart des Krebses in einem Testpatienten umfasst, der eine der sechs Krebsarten aufweist, wobei die Unterartenklassifizierung die folgenden Schritte umfasst:
(a) Verwenden eines kontrollierten Algorithmus, um Genexpressionszentroide für jede der Krebsunterarten in dem Trainingssatz zu konstruieren;
(b) Berechnen der Entfernung des Genexpressionsprofils der Testprobe zu jedem der Glycosyltransferase-Genexpressionszentroide, die in Schritt (a) konstruiert wurden; und
(c) Zuweisen der Testprobe zu einer Krebsunterart, wobei die Krebsunterart als jene bestimmt ist, die dem nächsten Trainingssatz-Glycosyltransferase-Genzentroid im Vergleich mit dem Glycosyltransferase-Genexpressionsprofil aus der Testprobe entspricht.

7. Verfahren nach einem der Ansprüche 4 bis 6, wobei die Überlebensinformationen für jeden Satz von Referenz-Glycosyltransferase-Genexpressionsprofilen oder -zentroiden bekannt ist und die Überlebensprognose des Testpatienten auf Grundlage der Überlebensprognose des Unterarten-Clusters identifiziert ist, auf dem die Test-Glycosyltransferase-Genexpressionsprofilgruppen beruhen.

8. Verfahren nach einem der Ansprüche 4 bis 7, wobei der Schritt des Einordnens in eine Unterart des Krebses nach Identifizierung der Krebsart an Proben von Patienten durchgeführt wird, die als Brust-, Hirn- oder Ovarialkrebs aufweisend identifiziert wurden.

9. Verfahren nach einem der vorhergehenden Ansprüche, wobei der Schritt des Erhaltens eines Glycosyltransferase-Genexpressionsprofils ferner den folgenden Schritt umfasst:
(a) Reinigen der gesamten RNA aus der Probe des Testpatienten;
(b) Umwandeln der RNA in cDNA durch reverse Transkription und Kennzeichnen der cDNA mit einem fluoreszenten Farbmittel oder Verstärken von mRNA aus der RNA und Kennzeichnen der mRNA mit einem fluoreszenten Farbmittel;
(c) Berühren eines Mikroarray-Objektträgers, der mit Ziel-Oligonukleotidsonden bedruckt ist, die für die 210 Glycosyltransferase-Gene, die in Tabelle 1 aufgelistet sind, oder ein Genfragment von jedem davon spezifisch sind, mit der fluoreszent gekennzeichneten cDNA oder mRNA;
(d) Hybridisieren der fluoreszent gekennzeichneten cDNA oder mRNA mit den Ziel-Oligonukleotidsonden; und
(e) Quantifizieren der Intensität der Fluoreszenz von cDNA oder mRNA, die an ihre Ziel-Oligonukleotidsonden auf dem Mikroarray-Objektträger gebunden sind, wobei die Intensität der Fluoreszenz an jedem Ziel der relativen Menge von mRNA-Transkript für jedes Patienten-Glycosyltransferase-Gen entspricht und das Patientenproben-Glycosyltransferase-Genexpressionsprofil ist.

10. Verfahren nach einem der Ansprüche 1 bis 8, wobei der Schritt des Erhaltens eines Glycosyltransferase-Genexpressionsprofils die folgenden Schritte umfasst:
(a) Reinigen der gesamten RNA aus der Probe des Testpatienten;
(b) Umwandeln der RNA in cDNA durch reverse Transkription und Kennzeichnen der cDNA mit einem fluoreszenten Farbmittel oder einem chromogenen Substrat oder Verstärken von mRNA aus der RNA und Kennzeichnen der mRNA mit einem fluoreszenten Farbmittel oder einem chromogenen Substrat;
(c) Berühren eines Dot-Blot, der mit Ziel-Oligonukleotidsonden bedruckt ist, die für die 210 Glycosyltransferase-Gene, die in Tabelle 1 aufgelistet sind, oder ein Genfragment von jeder der gekennzeichneten cDNA oder mRNA spezifisch sind;
(d) Hybridisieren der gekennzeichneten cDNA oder mRNA mit den Ziel-Oligonukleotidsonden; und
(e) Quantifizieren der Intensität der hybridisierten gekennzeichneten cDNA oder mRNA, die an ihre Ziel-Oligonukleotidsonden auf dem Dot-Blot gebunden sind, wobei die Intensität der Kennzeichnung an jedem Ziel der relativen Menge von mRNA-Transkript für jedes Gen entspricht und das Patientenproben-Glycosyltransferase-Genexpressionsprofil ist.

11. Verfahren nach einem der Ansprüche 1 bis 8, wobei der Schritt des Erhaltens eines Glycosyltransferase-Genexpressionsprofils ferner die folgenden Schritte umfasst:
(a) Reinigen der gesamten RNA aus der Probe des Testpatienten;
(b) Verstärken der cDNA aus der RNA durch RT-qPCR mit spezifischen Primern für die 210 Glycosyltransferase-Gene, die in Tabelle 1 aufgelistet sind, oder ein Genfragment von jedem;
(c) Quantifizieren der verstärkten cDNA; und
(d) Bewerten der relativen Menge der verstärkten cDNA von jedem der Gene, wobei die Menge der verstärkten cDNA der relativen Menge der mRNA-Transkripts für jedes Gen entspricht und das Patientenproben-Glycosyltransferase-Genexpressionsprofil ist.

12. Kit zum Klassifizieren einer Krebsart, die ausgewählt ist aus der Gruppe bestehend aus sechs Krebsarten ausgewählt aus invasivem Brustkarzinom, Plattenepithelkarzinom der Lunge und serösem Ovarialcystadenokarzinom, Glioblastoma multiforme, Dickdarm-Adenokarzinom und Klarzellen-Nierenkarzinom in einer Testprobe eines Patienten und gegebenenfalls ebenfalls einer Krebsunterart in dem Patienten unter Verwendung des Verfahrens nach einem der Ansprüche 1 bis 11, wobei das Kit aus Folgendem besteht:
(a) Primern zur Verstärkung von cDNA oder mRNA; oder
(b) genspezifischen Oligonukleotidsonden zum Hybridisieren mit cDNA oder mRNA in einem Dot-Blot-Vorgang; oder
(c) genspezifischen Oligonukleotidsonden, die auf einen Mikroarray-Objektträger gedruckt sind, mit denen gekennzeichnete cDNA oder RNA in einem Mikroarray-Verfahren hybridisiert werden kann; und
(d) einem Indikatormittel;
wobei die Primer oder genspezifische Oligonukleotidsonden für die 210 Glycosyltransferase-Gene oder Fragmente davon, die in Tabelle 1 aufgelistet sind, spezifisch sind;
(e) gegebenenfalls eine Gebrauchsanweisung;
(f) gegebenenfalls einem Referenzsatz von Glycosyltransferase-Polynukleotiden der sechs Krebsarten oder Unterarten davon, wobei die Polynukleotide die Glycosyltransferase-Gene, die in Tabelle 1 aufgelistet sind, oder Fragmente davon umfassen; und
(g) gegebenenfalls computerlesbaren Anweisungen für beliebige oder mehr der Folgenden:
A. Bestimmen des Glycosyltransferase-Genexpressionsprofils der Patiententestprobe;
B. Identifizieren einer Krebsart durch:
i. Berechnen der Entfernung des Genexpressions-Profils der Patiententestprobe zu jedem des Satzes von Glycosyltransferase-Genexpressionszentroiden der sechs Krebsarten; und
ii. Zuweisen der Patiententestprobe einer Krebsart, wobei die Krebsart als jene bestimmt ist, die dem nächsten Glycosyltransferase-Genexpressionszentroid entspricht, im Vergleich mit dem Glycosyltransferase-Genexpressionsprofil aus der Patiententestprobe; und
C. Klassifizieren und/oder Identifizieren der Krebsunterart durch Folgendes:
i. Durchführen unkontrollierter Konsens-Cluster-Analyse des Glycosyltransferase-Genexpressionsprofils aus der Testprobe, die eine der sechs Krebsarten aufweist, im Vergleich mit den Glycosyltransferase-Genexpressionsprofilen aus einem Satz von Referenz- oder Trainings-Glycosyltransferasegenen aus Unterarten des entsprechenden Krebses mit jenen des Patienten;
ii. Berechnen der Entfernung des Genexpressionsprofils der Patiententestprobe mit jedem des Satzes von Glycosyltransferase-Genexpressionszentroiden aus Unterarten des entsprechenden Krebses zu jenem des Patienten; und
iii. Zuweisen der Patiententestprobe zu einer Krebsunterart, wobei die Krebsunterart als jene bestimmt ist, die dem nächsten Glycosyltransferase-Genexpressionszentroid entspricht, im Vergleich mit dem Glycosyltransferase-Genexpressionsprofil aus der Patiententestprobe.

13. Kit nach Anspruch 12, wobei die Überlebensinformationen für jeden Satz von Referenz- oder Trainings-Glycosyltransferase-Genexpressionsprofilen oder -zentroiden bekannt ist, das ferner computerlesbare Anweisungen zur Identifizierung der Überlebensprognose des Patienten auf Grundlage der Überlebensprognose des Unterart-Clusters umfasst, innerhalb welchem sich das Test-Glycosyltransferase-Genexpressionsprofil gruppiert.

14. Verfahren zur Auswahl eines Behandlungsschemas für einen Patienten, von dem ausgegangen wird oder von dem bekannt ist, dass es Krebs aufweist, das Durchführen des Verfahrens nach einem der Ansprüche 1 bis 11 an einer Probe des Patienten und dann auswählen des geeigneten Behandlungsschemas für die Krebsart oder -unterart, die in dem Patienten identifiziert ist, umfasst, wobei das Verfahren durchgeführt wird, bevor der Patient einer therapeutischen Behandlung für Krebs unterzogen wird, und dann nochmals nachdem der Patient einer therapeutischen Behandlung für Krebs unterzogen wurde, wobei eine Differenz in den Glycosyltransferase-Genexpressionspegeln von vor der Behandlung zu nach der Behandlung die Wirksamkeit der therapeutischen Behandlung angibt.

## Revendications

1. Procédé *in vitro* d'identification d'un type de cancer appartenant au groupe constitué de six types de cancer choisis parmi le carcinome invasif du sein, le carcinome épidermoïde du poumon, le cancer du cystadénocarcinome séreux ovarien, le glioblastome multiforme, l'adénocarcinome du côlon et le carcinome à cellules claires du rein, dans un échantillon étudié provenant d'un sujet comprenant les étapes :
(a) d'obtention d'un profil d'expression du gène de la glycosyltransférase provenant d'un ensemble de 210 gènes de la glycosyltransférase constitué de ceux énumérés dans le tableau 1 dans l'échantillon étudié provenant du sujet ;
(b) de calcul de la distance du profil d'expression génétique de l'échantillon étudié à un ensemble de référence de centroïdes d'expression du gène de la glycosyltransférase provenant des six types de cancer ; et
(c) d'assignation de l'échantillon étudié à un type de cancer, dans lequel le type de cancer est identifié comme étant le type de cancer correspondant au centroïde du gène de la glycosyltransférase de l'ensemble de référence le plus proche des centroïdes d'expression du gène de la glycosyltransférase des six types de cancer par rapport au profil d'expression du gène de la glycosyltransférase provenant de l'échantillon étudié.

2. Procédé selon la revendication 1, comprenant les étapes suivantes :
(a) l'obtention d'un profil d'expression du gène de la glycosyltransférase pour chacun d'un ensemble d'apprentissage d'échantillons de cancer provenant de sujets présentant chacun des six types de cancer, dans lequel les gènes de la glycosyltransférase sont constitués des 210 gènes de la glycosyltransférase énumérés dans le tableau 1 ;
(b) l'utilisation d'un algorithme supervisé pour construire des centroïdes d'expression génique pour chacun des six types de cancer dans l'ensemble d'apprentissage ;
(c) le calcul de la distance du profil d'expression génétique de l'échantillon étudié à chacun des centroïdes d'expression du gène de la glycosyltransférase provenant de l'ensemble d'apprentissage des six types de cancer ; et
(d) l'assignation de l'échantillon étudié à un type de cancer, dans lequel le type de cancer est identifié comme étant celui correspondant au centroïde du gène de la glycosyltransférase de l'ensemble d'apprentissage le plus proche des six types de cancer par rapport au profil d'expression du gène de la glycosyltransférase provenant de l'échantillon étudié.

3. Procédé selon la revendication 1 ou 2, dans lequel l'ensemble de référence ou l'ensemble d'apprentissage des types de cancer, ou les deux, est constitué des types de cancer choisis parmi le cancer du sein, y compris le cancer invasif du sein, le cancer du cerveau, y compris le glioblastome multiforme, le cancer du côlon, y compris l'adénocarcinome du côlon, le cancer du rein, y compris le carcinome à cellules claires du rein, le cancer du poumon, y compris le carcinome épidermoïde du poumon, et le cancer des ovaires, y compris le cancer du cystadénocarcinome séreux ovarien.

4. Procédé selon l'une quelconque des revendications précédentes, dans lequel le procédé comprend en outre une étape de classification du sous-type du cancer chez un sujet étudié présentant l'un des six types de cancer, dans lequel la classification par sous-type comprend les étapes :
(a) de fourniture d'un ensemble de référence de profils d'expression du gène de la glycosyltransférase provenant de sous-types du type de cancer correspondant à celui du sujet, dans lequel les gènes de la glycosyltransférase sont constitués des 210 gènes de la glycosyltransférase énumérés dans le tableau 1 ;
(b) de réalisation d'une analyse de regroupement non supervisée sur le profil d'expression du gène de la glycosyltransférase du sujet étudié et l'ensemble de référence des profils d'expression du gène de la glycosyltransférase ; et
(c) de classification du sous-type du sujet étudié en regroupant le profil d'expression du gène de la glycosyltransférase étudié en un ou plusieurs sous-types correspondant au profil d'expression du gène de la glycosyltransférase de référence le plus proche pour un sous-type.

5. Procédé selon l'une quelconque des revendications précédentes, dans lequel le procédé comprend en outre une étape de classification du sous-type du cancer chez un sujet étudié présentant l'un des six types de cancer, dans lequel la classification par sous-type comprend les étapes :
(a) de calcul de la distance du profil d'expression génétique de l'échantillon étudié à chacun d'un ensemble de centroïdes d'expression du gène de la glycosyltransférase de référence provenant des sous-types du cancer correspondant à celui du sujet ; et
(b) d'assignation de l'échantillon étudié à un sous-type de cancer, dans lequel le sous-type de cancer est identifié comme étant celui correspondant au centroïde du gène de la glycosyltransférase de l'ensemble de référence le plus proche par rapport au profil d'expression du gène de la glycosyltransférase provenant de l'échantillon étudié.

6. Procédé selon l'une quelconque des revendications 2, 3 ou 5, dans lequel le procédé comprend en outre une étape de classification du sous-type du cancer chez un sujet étudié présentant l'un des six types de cancer, dans lequel la classification par sous-type comprend les étapes :
(a) d'utilisation d'un algorithme supervisé pour construire des centroïdes d'expression génique pour chacun des sous-types de cancer dans l'ensemble d'apprentissage ;
(b) de calcul de la distance du profil d'expression génétique de l'échantillon étudié à chacun des centroïdes d'expression du gène de la glycosyltransférase construit dans l'étape (a) ; et
(c) d'assignation de l'échantillon étudié à un sous-type de cancer, dans lequel le sous-type de cancer est déterminé comme étant celui correspondant au centroïde du gène de la glycosyltransférase de l'ensemble d'apprentissage le plus proche par rapport au profil d'expression du gène de la glycosyltransférase provenant de l'échantillon étudié.

7. Procédé selon l'une quelconque des revendications 4 à 6, dans lequel des informations de survie pour chacun de l'ensemble de profils ou de centroïdes d'expression du gène de la glycosyltransférase de référence sont connues et le pronostic de survie du sujet étudié est identifié sur la base du pronostic de survie du regroupement de sous-types dans lequel se trouve le profil d'expression du gène de la glycosyltransférase étudié.

8. Procédé selon l'une quelconque des revendications 4 à 7, dans lequel l'étape de sous-typage du cancer après identification du type de cancer est réalisée sur des échantillons provenant de sujets identifiés comme ayant un cancer du sein, du cerveau ou des ovaires.

9. Procédé selon l'une quelconque des revendications précédentes, dans lequel l'étape d'obtention d'un profil d'expression du gène de la glycosyltransférase comprend en outre les étapes :
(a) de purification de l'ARN total provenant de l'échantillon du sujet étudié ;
(b) de conversion de l'ARN en ADNc par transcription inverse et de marquage de l'ADNc avec un colorant fluorescent ou d'amplification de l'ARNm provenant de l'ARN et de marquage de l'ARNm avec un colorant fluorescent ;
(c) de mise en contact d'une lame de microréseau imprimée avec des sondes oligonucléotidiques cibles spécifiques des 210 gènes de la glycosyltransférase énumérés dans le tableau 1 ou d'un fragment de gène de chacun d'eux avec l'ADNc ou l'ARNm marqué par fluorescence ;
(d) d'hybridation de l'ADNc ou de l'ARNm marqué par fluorescence avec les sondes oligonucléotidiques cibles ; et
(e) de quantification de l'intensité de la fluorescence de l'ADNc ou de l'ARNm liée à leurs sondes oligonucléotidiques cibles sur la lame de microréseau, dans lequel l'intensité de la fluorescence au niveau de chaque cible correspond à l'abondance relative du transcrit d'ARNm pour chaque gène de la glycosyltransférase du sujet et constitue le profil d'expression du gène de la glycosyltransférase de l'échantillon du sujet.

10. Procédé selon l'une quelconque des revendications 1 à 8, dans lequel l'étape d'obtention d'un profil d'expression du gène de la glycosyltransférase comprend les étapes :
(a) de purification de l'ARN total provenant de l'échantillon du sujet étudié ;
(b) de conversion de l'ARN en ADNc par transcription inverse et de marquage de l'ADNc avec un colorant fluorescent ou avec un substrat chromogène, ou d'amplification de l'ARNm provenant de l'ARN et de marquage de l'ARNm avec un colorant fluorescent ou avec un substrat chromogène ;
(c) de mise en contact d'un transfert de points imprimé avec des sondes oligonucléotidiques cibles spécifiques des 210 gènes de glycosyltransférase énumérés dans le tableau 1 ou d'un fragment de gène de chacun d'eux avec l'ADNc ou l'ARNm marqué ;
(d) d'hybridation de l'ADNc ou de l'ARNm marqué avec les sondes oligonucléotidiques cibles ; et
(e) de quantification de l'intensité de l'ADNc ou de l'ARNm marqué hybridé liée à leurs sondes oligonucléotidiques cibles sur le transfert de points, dans lequel l'intensité de l'étiquette au niveau de chaque cible correspond à l'abondance relative du transcrit d'ARNm pour chaque gène et constitue le profil d'expression du gène de la glycosyltransférase de l'échantillon du sujet.

11. Procédé selon l'une quelconque des revendications 1 à 8, dans lequel l'étape d'obtention d'un profil d'expression du gène de la glycosyltransférase comprend en outre les étapes :
(a) de purification de l'ARN total provenant de l'échantillon du sujet étudié ;
(b) d'amplification de l'ADNc provenant de l'ARN par RT-qPCR avec des amorces spécifiques pour les 210 gènes de glycosyltransférase énumérés dans le tableau 1 ou d'un fragment de gène de chacun d'eux ;
(c) de quantification de l'ADNc amplifié ; et
(d) d'évaluation de l'abondance relative de l'ADNc amplifié de chacun des gènes, dans lequel l'abondance de l'ADNc amplifié correspond à l'abondance relative du transcrit d'ARNm pour chaque gène et constitue le profil d'expression du gène de la glycosyltransférase de l'échantillon du sujet.

12. Kit de classification d'un type de cancer choisi dans le groupe constitué de six types de cancer choisis parmi le carcinome invasif du sein, le carcinome épidermoïde du poumon et le cancer du cystadénocarcinome séreux ovarien, le glioblastome multiforme, l'adénocarcinome du côlon et le carcinome à cellules claires du rein, dans un échantillon étudié provenant d'un sujet et éventuellement aussi d'un sous-type de cancer chez le sujet en utilisant le procédé selon l'une quelconque des revendications 1 à 11, le kit consistant en :
(a) des amorces pour l'amplification d'ADNc ou d'ARNm ; ou
(b) des sondes oligonucléotidiques spécifiques d'un gène pour l'hybridation à un ADNc ou à un ARNm dans une procédure de transfert de points ; ou
(c) des sondes oligonucléotidiques spécifiques d'un gène imprimées sur une lame de microréseau, auxquelles un ADNc ou un ARN marqué peut être hybridé dans un procédé de microréseau ; et
(d) un moyen indicateur ;
dans lequel les amorces ou les sondes oligonucléotidiques spécifiques d'un gène sont spécifiques aux 210 gènes de glycosyltransférase ou à leurs fragments énumérés dans le tableau 1 ;
(e) éventuellement, un mode d'emploi ;
(f) éventuellement un ensemble de référence de polynucléotides de glycosyltransférase provenant des six types de cancer ou de leurs sous-types, dans lequel les polynucléotides comprennent les gènes de la glycosyltransférase énumérés dans le tableau 1 ou des fragments de ceux-ci ; et
(g) éventuellement des instructions lisibles par ordinateur pour un quelconque ou plusieurs parmi :
A. la détermination du profil d'expression du gène de la glycosyltransférase de l'échantillon étudié du sujet ;
B. l'identification du type de cancer par :
i. calcul de la distance du profil d'expression génétique de l'échantillon étudié du sujet à chacun de l'ensemble des centroïdes d'expression du gène de la glycosyltransférase provenant des six types de cancer ; et
ii. assignation de l'échantillon étudié du sujet au type de cancer, dans lequel le type de cancer est déterminé comme étant celui correspondant au centroïde d'expression du gène de la glycosyltransférase le plus proche par rapport au profil d'expression du gène de la glycosyltransférase provenant de l'échantillon étudié du sujet ; et
C. la classification et/ou l'identification du sous-type de cancer par :
i. réalisation d'une analyse de regroupement par consensus non supervisée du profil d'expression du gène de la glycosyltransférase provenant de l'échantillon étudié du sujet présentant l'un des six types de cancer comparé aux profils d'expression du gène de la glycosyltransférase provenant d'un ensemble de gènes de la glycosyltransférase de référence ou d'apprentissage provenant de sous-types du cancer correspondant à celui du sujet ;
ii. calcul de la distance du profil d'expression génétique de l'échantillon étudié du sujet à chacun de l'ensemble de centroïdes d'expression du gène de la glycosyltransférase provenant de sous-types du cancer correspondant à celui du sujet ; et
iii. assignation de l'échantillon étudié du sujet à un type de cancer, dans lequel le sous-type de cancer est déterminé comme étant celui correspondant au centroïde d'expression du gène de la glycosyltransférase le plus proche par rapport au profil d'expression du gène de la glycosyltransférase provenant de l'échantillon étudié du sujet.

13. Kit selon la revendication 12, dans lequel des informations de survie pour chacun de l'ensemble de profils ou de centroïdes d'expression du gène de la glycosyltransférase de référence ou d'apprentissage sont connues, comprenant en outre des instructions lisibles par ordinateur pour l'identification du pronostic de survie du sujet sur la base du pronostic de survie du regroupement de sous-types dans lequel se trouve le profil d'expression du gène de la glycosyltransférase étudié.

14. Procédé de sélection d'un régime de traitement pour un sujet soupçonné d'avoir eu ou connu comme ayant un cancer, comprenant la réalisation du procédé selon l'une quelconque des revendications 1 à 11 sur un échantillon provenant du sujet puis de sélection du régime de traitement approprié pour le type ou le sous-type de cancer identifié chez le sujet, dans lequel le procédé est réalisé avant que le sujet subisse un traitement thérapeutique contre le cancer, puis à nouveau après que le sujet ait subi un traitement thérapeutique contre le cancer, dans lequel une différence entre les niveaux d'expression du gène de la glycosyltransférase allant d'avant le traitement à après le traitement est révélatrice de l'efficacité du traitement thérapeutique.
